# EUROPEAN PATENT APPLICATION

(11) **EP 4 177 337 A1**
(43) Date of publication of application: **10.05.2023**
(21) Application number: 21834496.8
(22) Date of filing: 01.07.2021
(51) Int. Cl.: C12N 5/071, A61K 35/28, A61P 3/00, A61P 3/06, A61P 7/00, A61P 31/18, A61P 35/00, A61P 43/00, C12N 5/10, C12Q 1/02, G01N 33/53

(54) **HUMAN LONG-TERM HEMATOPOIETIC STEM CELL MARKER**

(30) Priority: 01.07.2020 WO PCT/JP2020/025915; 27.05.2021 JP 2021089502
(71) Applicant: NEXTGEM INC., Tokyo 150-0041 (JP)
(72) Inventor: MIYANISHI Masanori, Tokyo 141-0021 (JP); MIYATSUKA Isao, Tokyo 141-0021 (JP); Le My An, Tokyo 141-0021 (JP)
(74) Representative: Nederlandsch Octrooibureau
(86) International application number: PCT/JP2021/025050
(87) International publication number: WO 2022/004864

(57) **Abstract**

The present disclosure relates to a marker for enriching or isolating hematopoietic stem cells and a method for using the same, cells or a cell population isolated or enriched by the marker, a method for using the cells or cell population, a drug containing the cells or cell population, and a classifier obtained using the cells or cell population.

## Description

### Technical Field

The present disclosure relates to a marker for enriching or isolating hematopoietic stem cells and a method for using the same, cells or a cell population isolated or enriched by the marker, a method for using the cells or cell population, a drug containing the cells or cell population, and a classifier obtained using the cells or cell population.

### Background Art

A stem cell, for example, a hematopoietic stem cell (HSC), is known to have biological functions of self-renewal ability and pluripotency. Among stem cells having self-renewal ability and pluripotency, it is known that there are cells that maintain a biological function characterizing these stem cells for a long period of time and stem cells that lose the function in a relatively short period of time (Non Patent Literature 1).

Stem cells that maintain self-renewal ability and pluripotency for a long period of time, for example, for a lifetime of an individual organism, can be mass-cultured using their self-renewal ability, and can be differentiated into various cells using their pluripotency. Stem cells having such characteristics are considered to be cells that can serve as a trump card when regenerative medicine is put to practical use. However, all of these stem cells are cells whose abundance in a living body is extremely rare, and cannot be easily identified and isolated from a living body.

A hematopoietic stem cell (HSC) is defined as a blood cell that exists mainly in the bone marrow and has self-renewal ability and multipotency, and is used for hematopoietic stem cell transplantation and the like. When high-quality hematopoietic stem cells such as long-term hematopoietic stem cells that maintain characteristics of stem cells for a long period of time can be appropriately isolated, although it is considered that the hematopoietic stem cells can be proliferated in vitro in large quantities, no specific marker was known to allow appropriate isolation of long-term hematopoietic stem cells (long-term HSCs: LT-HSCs) in a human.

Conventionally, attempts have been made to identify molecules specifically expressed on cell surfaces of hematopoietic stem cells and to identify hematopoietic stem cells having specific characteristics by a combination of these molecules. However, in such a technique, it is required to find an "optimal combination of monoclonal antibodies" for identifying hematopoietic stem cells having specific characteristics through trial and error, and a highly skilled technique has been required. In addition, due to a large number of combinations of monoclonal antibodies to be tested, such a development technique generally requires a huge amount of time and cost. In fact, since the discovery of mouse hematopoietic stem cells (HSCs) in 1988, it took as much as 30 years to discover and identify, in particular, long-term hematopoietic stem cells that maintain self-renewal ability and multipotency for a lifetime of a mouse individual, among cells having characteristics of hematopoietic stem cells (Non Patent Literature 2). On the other hand, it has been suggested that molecular markers specifically expressed on the surfaces of the hematopoietic stem cells are different between the mouse and the human, and it has been desired to find a marker capable of enriching and identifying long-term hematopoietic stem cells in the human.

In order to identify and isolate long-term hematopoietic stem cells, attempts have been made so far to search for combinations of molecules specifically expressed on the surfaces of the long-term hematopoietic stem cells. For example, in order to further enrich hematopoietic stem cells from a CD34-positive hematopoietic stem cell population, it has been proposed to isolate a Thy1-positive fraction from CD34-positive, CD38-negative, and CD45RAnegative fractions in cord blood, but it has been reported that the fraction still contains various cells that differ in the ability to maintain the characteristics of the hematopoietic stem cells (Non Patent Literature 3). Searching for such combinations of cell surface molecules generally requires trial and error involving a large amount of work for performing a cell function test (Non Patent Literature 4). Therefore, it is desired to identify novel combinations of cell surface molecules to identify long-term hematopoietic stem cells.

### Citation List

### Non Patent Literatures

Non Patent Literature 1: Japanese Journal of Transfusion and Cell Therapy, 2006, Vol. 52, No. 5, pp. 583-588
Non Patent Literature 2: nature, 2016, Vol. 530, pp. 223-227
Non Patent Literature 3: Science, 2011, Vol. 333, pp. 218-221
Non Patent Literature 4: Cell Stem Cell, 2007, Vol. 1, pp. 635-645

### Summary of Invention

### Technical Problem

An object of the present disclosure is to provide a marker for enriching or isolating hematopoietic stem cells and a use thereof.

### Solution to Problem

In one aspect, the present disclosure provides a long-term hematopoietic stem cell isolation marker or enrichment marker containing one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof.

In one aspect, the present disclosure provides isolated hematopoietic stem cells or a hematopoietic stem cell-containing cell population in which hematopoietic stem cells are enriched that is identified by an expression pattern of one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof.

In one aspect, the present disclosure provides a method for producing isolated or enriched hematopoietic stem cells or a cell population containing hematopoietic stem cells, the method including:
preparing a cell population containing hematopoietic stem cells; and
isolating or enriching cells using, as an index, an expression pattern of one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof.

In another aspect, the present disclosure provides a method for evaluating a content and/or a change in content of hematopoietic stem cells in the cell population, the method including:
preparing a cell population containing hematopoietic stem cells; and
measuring, in each cell, an expression level of a marker containing one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48 of each cell, or one or more selected from a combination of CD48 and CD34, a combination of CD48 and c-Kit, and a combination of CD48, CD34, and c-Kit, or a combination thereof.

In another aspect, the present disclosure provides a test method for a cell population containing hematopoietic stem cells, the test method including:
preparing a cell population containing hematopoietic stem cells; and
testing quality of the cell population using, as an index, an expression level of a marker containing one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in the cells contained in the cell population containing hematopoietic stem cells.

In another aspect, the present disclosure provides a test method for a cell population containing hematopoietic stem cells, the test method including:
preparing a cell population containing hematopoietic stem cells; and
testing transplantation compatibility of the cell population using, as an index, an expression level of a marker containing one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in the cells contained in the cell population containing hematopoietic stem cells.

In another aspect, the present disclosure provides a test method for a cell population containing hematopoietic stem cells, the test method including:
for predicting a disease prognosis or a health status of a subject,
obtaining an expression level of a marker containing one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in cells contained in a cell population containing patientderived hematopoietic stem cells; and
comparing the expression level of the marker with a control.

In another aspect, the present disclosure provides a method for screening a compound modifying characteristics of hematopoietic stem cells, the method including:
preparing a cell population containing hematopoietic stem cells;
treating the cell population containing hematopoietic stem cells with a candidate compound; and
selecting a candidate compound using, as an index, an expression level of a marker containing one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in the cells treated with the candidate compound.

In another aspect, the present disclosure provides a method for adjusting culture conditions to modify characteristics of hematopoietic stem cells, the method including:
preparing a cell population containing hematopoietic stem cells;
culturing the cell population containing hematopoietic stem cells under adjusted culture conditions; and
determining a method for adjusting culture conditions using, as an index, an expression level of a marker containing one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in the cells cultured under the adjusted culture conditions.

In another aspect, the present disclosure provides a method for producing isolated or enriched hematopoietic stem cells or a cell population containing hematopoietic stem cells, the method including:
preparing a cell population containing hematopoietic stem cells; and
adjusting culture conditions to modify characteristics of the hematopoietic stem cells using, as an index, an expression level of one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48 that are expressed in the cells contained in the cell population, or one or more selected from a combination of CD48 and CD34, a combination of CD48 and c-Kit, and a combination of CD48, CD34, and c-Kit, or a combination thereof.

In another aspect, the present disclosure provides a method for producing a culture medium or a culture auxiliary additive that modifies characteristics of hematopoietic stem cells, the method including:
preparing a cell population containing hematopoietic stem cells;
culturing the cell population containing hematopoietic stem cells in a medium having a configuration to be evaluated or a medium containing a culture auxiliary additive to be evaluated;
determining a configuration of the culture medium or the culture auxiliary additive using, as an index, an expression level of a marker containing one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in the cultured cells; and
producing a culture medium or a culture auxiliary additive that has the determined configuration.

In another aspect, the present disclosure provides a method for screening a compound inducing hematopoietic stem cells, the method including:
preparing a cell population;
treating the cell population with a test compound; and
selecting a candidate compound using, as an index, an expression level of a marker containing one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in the cells treated with a candidate compound.

In another aspect, the present disclosure provides a composition or kit for identifying, detecting, isolating, or enriching hematopoietic stem cells, the composition or kit containing a reagent for quantifying or detecting an expression level of a marker containing one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof.

In another aspect, the present disclosure provides a method of producing modified hematopoietic stem cells by modifying the hematopoietic stem cells of the present disclosure.

In another aspect, the present disclosure provides a drug for preventing or treating a disease or disorder ameliorated or prevented by administering hematopoietic stem cells, the drug containing isolated or enriched hematopoietic stem cells, a cell population containing hematopoietic stem cells, or modified cells thereof.

In another aspect, the present disclosure provides a method for evaluating a physiological action of a test substance, the method including bringing the test substance into contact with isolated or enriched hematopoietic stem cells, a cell population containing hematopoietic stem cells, or modified cells thereof.

In another aspect, the present disclosure provides a method for identifying hematopoietic stem cells by a combination of physical, chemical, and biological characteristics obtained by analyzing isolated or enriched hematopoietic stem cells or a cell population containing hematopoietic stem cells, and producing a cell population containing the hematopoietic stem cells.

In another aspect, the present disclosure provides a method for producing a classifier for identifying hematopoietic stem cells by a combination of physical, chemical, and biological characteristics obtained by analyzing isolated or enriched hematopoietic stem cells or a cell population containing hematopoietic stem cells.

In another aspect, the present disclosure provides a drug for preventing or treating a disease or disorder ameliorated or prevented by administering hematopoietic stem cells, the drug containing cells produced using, as an index, an expression pattern of a marker molecule containing one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof.

In one aspect, the present invention provides the following.

### [Item 1]

A hematopoietic stem cell isolation marker or enrichment marker containing one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof.

### [Item 2]

The hematopoietic stem cell isolation marker or enrichment marker according to Item 1, in which the hematopoietic stem cell isolation marker or enrichment marker contains one or more selected from a combination of CD48 and CD34, a combination of CD48 and c-Kit, and a combination of CD48, CD34, and c-Kit.

### [Item 3]

Isolated hematopoietic stem cells or a hematopoietic stem cell-containing cell population in which hematopoietic stem cells are enriched that is identified by an expression pattern of one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof.

### [Item 4]

The isolated hematopoietic stem cells or the hematopoietic stem cell-containing cell population in which hematopoietic stem cells are enriched according to Item 3, in which the isolated hematopoietic stem cells or the hematopoietic stem cell-containing cell population in which hematopoietic stem cells are enriched is identified by an expression pattern of one or more selected from a combination of CD48(-) and CD34(+), a combination of CD48(-) and c-Kit(+), and a combination of CD48(-), CD34(+), and c-Kit(+).

### [Item 5]

A method for producing isolated or enriched hematopoietic stem cells or a cell population containing hematopoietic stem cells, the method including:
preparing a cell population containing hematopoietic stem cells; and
isolating or enriching cells using, as an index, an expression pattern of one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof.

### [Item 6]

The method for producing isolated or enriched hematopoietic stem cells or a cell population containing hematopoietic stem cells according to Item 5, in which
an expression pattern of one or more selected from a combination of CD48(-) and CD34(+), a combination of CD48(-) and c-Kit(+), and a combination of CD48(-), CD34(+), and c-Kit(+) is used as an index.

### [Item 7]

Hematopoietic stem cells identified, isolated, or enriched by the marker according to claim 1 or 2, the isolated or enriched hematopoietic stem cells or the cell population containing hematopoietic stem cells according to Item 3 or 4, or isolated or enriched hematopoietic stem cells or a cell population containing hematopoietic stem cells produced by the method according to Item 5 or 6, the hematopoietic stem cells or the cell population containing hematopoietic stem cells further containing a modification.

### [Item 8]

A method for evaluating a content and/or a change in content of hematopoietic stem cells in the cell population, the method including:
preparing a cell population containing hematopoietic stem cells; and
measuring, in each cell, an expression level of a marker containing one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48 of each cell, or one or more selected from a combination of CD48 and CD34, a combination of CD48 and c-Kit, and a combination of CD48, CD34, and c-Kit, or a combination thereof.

### [Item 9]

A test method for a cell population containing hematopoietic stem cells, the test method including:
preparing a cell population containing hematopoietic stem cells; and
testing quality of the cell population using, as an index, an expression level of a marker containing one or more selected from CD10, CD111, CD112, CD131, CD143, CD244, CD275, CD328, CD48, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in the cells contained in the cell population containing hematopoietic stem cells.

### [Item 10]

The test method according to Item 9, in which the quality is hematopoietic capacity maintenance ability, transplantation compatibility, or storage stability.

### [Item 11]

A test method for a cell population containing hematopoietic stem cells, the test method including:
obtaining an expression level of a marker containing one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in cells contained in a cell population containing hematopoietic stem cells derived from a subject; and
comparing the expression level of the marker with a control for predicting a disease prognosis or a health status of the subject.

### [Item 12]

A method for screening a compound modifying characteristics of hematopoietic stem cells, the method including:
preparing a cell population containing hematopoietic stem cells;
treating the cell population containing hematopoietic stem cells with a candidate compound; and
selecting a candidate compound using, as an index, an expression level of a marker containing one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in the cells treated with the candidate compound.

### [Item 13]

A method for adjusting culture conditions to modify characteristics of hematopoietic stem cells, the method including:
preparing a cell population containing hematopoietic stem cells;
culturing the cell population containing hematopoietic stem cells under adjusted culture conditions; and
determining culture conditions using, as an index, an expression level of a marker containing one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in the cells cultured under the adjusted culture conditions.

### [Item 14]

A method for producing a culture medium or a culture auxiliary additive that modifies characteristics of hematopoietic stem cells, the method including:
preparing a cell population containing hematopoietic stem cells;
culturing the cell population containing hematopoietic stem cells in a medium having a configuration to be evaluated or a medium containing a culture auxiliary additive to be evaluated;
determining a configuration of the culture medium or the culture auxiliary additive using, as an index, an expression level of a marker containing one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in the cultured cells; and
producing a culture medium or a culture auxiliary additive that has the determined configuration.

### [Item 15]

The method according to any one of Items 5 to 14, in which the cell population containing hematopoietic stem cells is cells derived from a living body or cells derived from cells having artificially acquired pluripotency.

### [Item 16]

Isolated or enriched hematopoietic stem cells or a cell population containing hematopoietic stem cells produced by the method according to any one of Items 5 to 7, in which the cell population containing hematopoietic stem cells is cells derived from a living body or cells derived from cells having artificially acquired pluripotency.

### [Item 17]

A method for screening a compound inducing hematopoietic stem cells, the method including:
preparing a cell population;
treating the cell population with a test compound; and
selecting a candidate compound using, as an index, an expression level of a marker containing one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in the cells treated with a candidate compound.

### [Item 18]

A composition or kit for identifying, detecting, isolating, or enriching hematopoietic stem cells, the composition or kit containing a reagent for quantifying or detecting an expression level of one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof.

### [Item 19]

A method for identifying a combination of physical, chemical, and biological characteristics obtained by analyzing hematopoietic stem cells identified, isolated, or enriched by the marker according to claim 1 or 2, the isolated or enriched hematopoietic stem cells or the cell population containing hematopoietic stem cells according to any one of Items 3, 4, and 7, or isolated or enriched hematopoietic stem cells or a cell population containing hematopoietic stem cells produced by the method according to Item 5 or 6.

### [Item 20]

A method for producing a classifier for identifying hematopoietic stem cells using, as an index, a combination of physical, chemical, and biological characteristics obtained by analyzing hematopoietic stem cells identified, isolated, or enriched by the marker according to claim 1 or 2, the isolated or enriched hematopoietic stem cells or the cell population containing hematopoietic stem cells according to any one of Items 3, 4, and 7, or isolated or enriched hematopoietic stem cells or a cell population containing hematopoietic stem cells produced by the method according to Item 5 or 6.

### [Item 21]

A drug for preventing or treating a disease or disorder ameliorated or prevented by administering hematopoietic stem cells, the drug containing hematopoietic stem cells identified, isolated, or enriched by the marker according to claim 1 or 2, the isolated or enriched hematopoietic stem cells or the cell population containing hematopoietic stem cells according to any one of Items 3, 4, and 7, or isolated or enriched hematopoietic stem cells or a cell population containing hematopoietic stem cells produced by the method according to Item 5 or 6.

### [Item 22]

The drug according to Item 21, in which the disease or disorder ameliorated or prevented by administering the hematopoietic stem cells is metachromatic leukodystrophy (MLD), Gaucher's disease, Fabry disease, mucopolysaccharidosis, HIV, ADA-SCID, X-SCID, or thalassemia.

### [Item 23]

The drug according to Item 21, in which the disease or disorder ameliorated or prevented by administering the hematopoietic stem cells is a chronic granulomatous disease (CGD) or adrenoleukodystrophy (ALD).

### Advantageous Effects of Invention

The present disclosure has an effect of providing a marker for enriching or isolating hematopoietic stem cells and a use thereof.

### Brief Description of Drawings

Fig. 1 is an image obtained by visualizing a cell arrangement spatial structure in which cells located close to each other on a differentiation lineage occupy positions close to each other, which is generated from data obtained by cell staining of a cord blood sample.
Fig. 2 is an image obtained by visualizing an expression level of CD34 in each cell in a cell arrangement spatial structure in which cells located close to each other on a differentiation lineage occupy positions close to each other, which is generated from data obtained by cell staining of a cord blood sample.
Fig. 3 is a view illustrating results of comparing chimera rates in a transplantation test of CD34(+) cells and CD34(-) cells obtained from cord blood.
Fig. 4 is an image showing a result of a cell arrangement in a CD34(+) fraction in more detail in the cell arrangement spatial structure in which cells located close to each other on a differentiation lineage occupy positions close to each other.
Fig. 5 is a view illustrating proliferation/differentiation potency of hematopoietic stem cells in a cord blood fraction identified by CD34(+) and CD38(-/+), and illustrates CFU assay results of a fraction (P6) composed of CD34(+) and CD38(+), a fraction (P7) composed of CD34(+), CD38(-), CD45RA(-), and CD90(+), a fraction (P8) composed of CD34(+), CD38(-), CD45RA(-), and CD90(-), and a fraction (P9) composed of CD34(+), CD38(-), CD45RA(+), and CD90(-).
Fig. 6 is a view illustrating an overview of the CFU assay at one-cell level.
Fig. 7 is a view illustrating a direction in which undifferentiated cells are located with respect to a CD34-positive cell fraction in the cell arrangement spatial structure in which cells located close to each other on a differentiation lineage occupy positions close to each other.
Fig. 8 is a view illustrating an example of a result of searching for a molecular marker whose expression is unevenly distributed in a differentiation direction in the cell arrangement spatial structure in which cells located close to each other on a differentiation lineage occupy positions close to each other.
Fig. 9 is a view illustrating a proportion of cells exhibiting CFU-GEMM (correlation with a degree of enrichment of undifferentiated cells) in the CFU assay of cells identified by each marker.
Fig. 10 is a view illustrating an example of a FACS plot when a cell population pre-enriched with CD34(+) from cord blood is gated on c-Kit(+) and CD34(+) (fraction P1) and is further gated on CD48(-) (P2 fraction) or CD48(+) (non-P2 fraction).
Fig. 11 is a view illustrating an example of a FACS plot when a cell population pre-enriched with CD34(+) from bone marrow is gated on c-Kit(+) and CD34(+) (fraction P1) and is further gated on CD48(-) (P2 fraction) or CD48(+) (non-P2 fraction).
Fig. 12 is a view illustrating an example of a FACS plot when a cell population pre-enriched with CD34(+) from mobilized peripheral blood is gated on c-Kit(+) and CD34(+) (fraction P1) and is further gated on CD48(-) (P2 fraction) or CD48(+) (non-P2 fraction).
Fig. 13 is a view illustrating a result of comparing the number of CD34-positive cells contained in per 1 ml of cord blood with the number of cells in the P2 fraction.
Fig. 14 is a view illustrating a result of comparing proliferation/differentiation potency of hematopoietic stem cells separated from cord blood in the cell fraction (P2) identified by c-Kit(+), CD34(+), and CD48(-) and the cell fraction (non-P2) identified by c-Kit(+), CD34(+), and CD48(+) by a CFU assay.
Fig. 15 is a view illustrating a result of comparing undifferentiation of a cell fraction P2 and a cell fraction non-P2 separated from cord blood by a transplantation test.
Fig. 16 is a view illustrating a result of comparing proliferation/differentiation potency of hematopoietic stem cells separated from bone marrow in the cell fraction (P2) identified by c-Kit(+), CD34(+), and CD48(-) and the cell fraction (non-P2) identified by c-Kit(+), CD34(+), and CD48(+) by a CFU assay, in which a result of the P1 fraction, which is a fraction identified by c-Kit(+) and CD34(+), is also illustrated.
Fig. 17 is a view illustrating a result of comparing proliferation/differentiation potency of hematopoietic stem cells separated from mobilized peripheral blood in the cell fraction (P2) identified by c-Kit(+), CD34(+), and CD48(-) and the cell fraction (non-P2) identified by c-Kit(+), CD34(+), and CD48(+) by a CFU assay, in which a result of the P1 fraction, which is a fraction identified by c-Kit(+) and CD34(+), is also illustrated.
Fig. 18 is a view illustrating a result of fractionating a CD34(+) fraction in the cord blood sample in a c-Kit(+) fraction and a c-Kit(-) fraction and comparing of proliferation/differentiation potency of hematopoietic stem cells in each fraction by a CFU assay, in which a result of the entire CD34(+) fraction is also illustrated.
Fig. 19 is a view illustrating an enrichment ratio of additional markers, in which data where the enrichment ratio exceeds 10 are illustrated as 10.
Fig. 20 is a view illustrating a result of culturing cells in the P2 fraction under a condition of addition or non-addition of UM171 and testing a total cell number and a ratio of undifferentiated cells (P1) after 7 days.
Fig. 21 is a view illustrating a result of culturing cells in the non-P2 fraction under a condition of addition or non-addition of UM171 and testing a total cell number and a ratio of undifferentiated cells (P1) after 7 days.
Fig. 22 illustrates a result of culturing cells in the P2 fraction fractionated from the cord blood sample with a marker set of c-Kit(+), CD34(+), and CD48(-) for 7 days, fractionating the cell population after culture in a P2 fraction (after culture) and a non-P2 fraction (after culture) using the marker set of c-Kit(+), CD34(+), and CD48(-), and performing a CFU assay for each fraction.

### Description of Embodiments

Hereinafter, embodiments of the present disclosure will be described. The following description is merely an example, and the scope of the present disclosure is not limited to the description, and can be appropriately changed and implemented without impairing the gist of the present disclosure.

### (Definition)

In the present disclosure, in a case where a plurality of ranges of numerical values are indicated, a range including any combination of a lower limit value and an upper limit value of the plurality of ranges is also meant in the same manner.

In the present disclosure, "differentiation" has a meaning commonly used in the art, and typically means a process by which cells showing a low degree of specialization are changed into more specialized cells, for example, nerve cells or muscle cells. "Differentiated" or "differentiating" is a relative term, and "differentiated cell" or "differentiating cell" means a cell more advanced and specialized in a developmental pathway than a cell to which it is being compared.

In the present disclosure, "stem cell" has a meaning commonly used in the art, and typically means an undifferentiated cell that has self-renewal ability at a single cell level and has ability to produce two or more different differentiated cells. That is, the stem cell can divide asymmetrically, and in this case, one daughter cell retains a stem cell state and the other daughter cell expresses a certain other specific biological function and phenotype. Alternatively, the stem cell can divide symmetrically into two stem cells, thus some stem cells are maintained in a cell population containing stem cells, while the other cells in the population only give rise to differentiated progeny.

In the present disclosure, "hematopoietic stem cell" has a meaning commonly used in the art, and typically means a cell having multipotency that can ultimately differentiate into all blood cells (red blood cells, white blood cells, platelets, and the like). The hematopoietic stem cell can encompass an intermediate stage of differentiation into progenitor cells or blast cells. The "progenitor cell" and "blast cell" are used interchangeably in the present disclosure, and mean maturing cells having reduced differentiation potential but still capable of maturing into different cells of a particular lineage (for example, bone marrow lineage or lymphoid lineage).

In the present disclosure, "long-term hematopoietic stem cell" has a meaning commonly used in the art, and typically means a hematopoietic stem cell that maintains self-renewal ability for a long period of time even after undergoing cell division. At least one of two daughter cells produced by cell division from long-term hematopoietic stem cells maintains a hematopoietic stem cell trait similar to that of the cell before division. In one aspect, the long-term hematopoietic stem cells do not lose any self-renewal ability by cell division in a normal culture environment in vivo or in a culture environment in vitro. In addition, in one aspect, the long-term hematopoietic stem cells maintain self-renewal ability even after undergoing 100, 50, 20, or 10 times of cell divisions.

The maintenance of the self-renewal ability can be confirmed by various known methods. For example, it can be confirmed that the self-renewal ability is maintained by measuring a content of an undifferentiated cell population contained in a cell group after culturing for a certain period of time. The undifferentiated cell population can be identified by, for example, a combination of known positive and negative markers expressed on a cell surface. In addition, in one aspect, it can be confirmed that the hematopoietic stem cells maintain the self-renewal ability for a long period of time even after undergoing cell division by maintaining bone marrow reconstitution ability for a long period of time. For example, long-term hematopoietic stem cells can maintain bone marrow reconstitution in a primary transplant recipient and also can reconstitute bone marrow in a secondary transplantation in a particularly preferred aspect in a known transplantation method. Hematopoietic stem cells that maintain self-renewal ability only for a short period of time can reconstitute bone marrow in vitro or in a primary transplantation, but cannot maintain bone marrow reconstitution in a primary transplant recipient, and cannot reconstitute and maintain bone marrow in a secondary transplantation. Here, the secondary transplantation means that hematopoietic stem cells derived from bone marrow reconstituted by the primary transplantation are transplanted to another individual. In one aspect, the fact that the hematopoietic stem cells maintain the self-renewal ability for a long period of time even after undergoing cell division can be confirmed by continuously producing blood for 4 days, 5 days, 6 days, 7 days, 8 days, 9 days, or 10 days or longer, and typically producing 500, 600, 700, 800, 900, or 1,000 cells/µL (mm³) or more of neutrophils, which is a type of white blood cells, for the period described above when the cells are transplanted to another living body.

Hematopoietic stem cells or long-term hematopoietic stem cells of the present disclosure encompass hematopoietic stem cells or long-term hematopoietic stem cells from various animals. For example, hematopoietic stem cells or long-term hematopoietic stem cells derived from various mammals, animals belonging to primates, animals belonging to simians, or humans can be used in the present disclosure, or the present disclosure can be applied to these cells. In addition, the hematopoietic stem cells of the present disclosure can be isolated from a somatic cell population obtained from a living body, and can also be isolated from a cell group induced to differentiate from ES cells or induced pluripotent stem cells.

In the present disclosure, "isolated cell" has a meaning commonly used in the art, and typically means a cell removed from an organism in which it is originally found or a progeny of such a cell. Such cells may be cultured in vitro, for example, in the presence of other cells. In addition, such cells or cells that are the progeny thereof may be later introduced into a second organism.

In the present disclosure, "isolated cell population" has a meaning commonly used in the art, and typically means a population of cells removed and separated from a heterogeneous cell population. In some aspects, the isolated population may be a substantially pure cell population as compared to the heterogeneous cell population from which the cells are isolated or enriched, or may still be a heterogeneous cell population.

"Substantially pure" of a cell population for a particular cell type has a meaning commonly used in the art, and typically means that in the cell population, at least about 50%, more preferably at least about 60%, 65%, 70%, 75%, 85%, 90%, 92%, or 93%, and most preferably at least about 95%, 96%, 97%, 98%, or 99% of the cells relative to the cells constituting the entire cell population are composed of cells of the particular cell type. For example, a "substantially pure" stem cell population means a population of cells containing less than about 50%, more preferably less than about 40%, 30%, 20%, 15%, 10%, 8%, or 7%, and most preferably less than about 5%, 4%, 3%, 2%, or 1% of cells that are not stem cells. Therefore, for example, the isolated hematopoietic stem cells in the present disclosure may be a cell population containing at least about 50%, more preferably at least about 60%, 65%, 70%, 75%, 85%, 90%, 92%, or 93%, and most preferably at least about 95%, 96%, 97%, 98%, or 99% of cells identified by a marker identified by the present disclosure.

In the present disclosure, "concentration" or "enrichment" has a meaning commonly used in the art, and typically means increasing a content ratio of organisms, cells, substances, data, and the like having a certain targeted characteristic in a population containing a plurality of organisms, cells, substances, data, and the like. For example, a marker for enriching long-term hematopoietic stem cells means that in a case where a cell population containing long-term hematopoietic stem cells is fractionated into two fractions by the marker, long-term hematopoietic stem cells are unevenly present in one of the fractions.

In the present disclosure, "identification", "separation", "sorting", "isolation", "purification", or "screening" has a meaning commonly used in the art, and typically refers to sorting of a target such as an organism, a cell, a substance, data, or the like that has certain targeted characteristics from a population containing a large number of cells by a specific manipulation/evaluation method. The sorted target may or may not be physically separated from the population. For example, a marker for isolating long-term hematopoietic stem cells means that in a case where a cell population containing long-term hematopoietic stem cells is fractionated into two fractions by the marker, long-term hematopoietic stem cells are unevenly present in one of the fractions at a certain level, and long-term hematopoietic stem cells present in the other fraction are below a certain level. For example, in a case where a cell population containing long-term hematopoietic stem cells is fractionated into fractions A and B, a value of long-term hematopoietic stem cells/(long-term hematopoietic stem cells present in fraction A + long-term hematopoietic stem cells present in fraction B) x 2 present in the fraction A is 1.4, 1.5, 1.6, 1.7, or 1.8 or more.

In the present disclosure, "promoting proliferation" of long-term hematopoietic stem cells has a meaning commonly used in the art, and typically means shortening the time required for cell division of long-term hematopoietic stem cells, increasing the number of long-term hematopoietic stem cells, or suppressing a decrease in content ratio of long-term hematopoietic stem cells associated with proliferation to maintain or increase the content ratio in a cell composition containing long-term hematopoietic stem cells and other cells.

### 1. Hematopoietic Stem Cell Marker of Present Disclosure

In general, various cells of a living body have unique states in various physical characteristics such as an intracellular content of a protein expressed from each gene, a type of protein exposed on a cell surface, a state of post-translational modification of a protein, and cell morphology, and it is possible to identify cells having unique biological functions by a combination of these physical characteristics. These physical characteristics can be quantified based on a certain standard by various known methods, and therefore various cells having a certain biological function can be identified by a combination of numerical data regarding the quantitative physical characteristics thereof. Furthermore, cells can be identified in more detail by combining a combination of the quantitative physical characteristic data and numerical data obtained from the analysis on biological characteristics.

On the other hand, cell differentiation has a hierarchy, and it is understood that cells that are more closely related in a cell differentiation hierarchy are also more similar in their quantitative physical characteristic data.

Therefore, a cell arrangement spatial structure is generated based on the similarity of a plurality of numerical data selected from numerical data on quantitative physical characteristics and/or numerical data on biological characteristics, such that it is possible to obtain a cell arrangement spatial structure in which cells located close to each other on a differentiation lineage occupy positions close to each other. Then, a differentiation direction in the cell arrangement spatial structure can be identified using a known differentiation marker or the like.

On the other hand, a region where cells of a specific differentiation stage exist is identified using a known differentiation marker or the like in the cell arrangement spatial structure configured as described above, such that a combination of ranges of numerical data of physical characteristics and/or biologically specific numerical data for identifying the cells of the differentiation stage is obtained.

The inventors of the present disclosure have succeeded in identifying a combination of marker molecules for identifying long-term hematopoietic stem cells having more undifferentiated cell characteristics extremely quickly with high accuracy by using analysis using the configured cell arrangement spatial structure and further combining cell function analysis at one-cell level.

Long-term hematopoietic stem cells having more undifferentiated cell characteristics of the present disclosure can be variously enriched or isolated using the marker depending on a desired accuracy. In one example, long-term hematopoietic stem cells having more undifferentiated cell characteristics of the present disclosure can be enriched or isolated by an expression level of a single marker molecule, and can be enriched or isolated by a combination of expression levels of a plurality of marker molecules in another example. In the present disclosure, in a case where cells are fractionated by an expression level of a specific marker molecule, a fraction having an expression level equal to or less than a certain level may be referred to as a "negative" fraction for the expression of the marker molecule, and a fraction having an expression level equal to or higher than a certain level may be referred to as a "positive" fraction for the expression of the marker molecule. A threshold for distinguishing "negative" and "positive" is usually determined by those skilled in the art according to an equipment to be used, a purpose of work, and the like. In one aspect, the cells can be fractionated based on a more detailed difference in expression level, and for example, the "positive" fraction can be further distinguished as a "weakly positive" fraction having a lower expression level and a "strongly positive" fraction having a lower expression level among the expression levels of the marker molecules. Long-term hematopoietic stem cells are enriched or isolated based on a classification of an expression level of a single marker molecule as "negative", "positive", "weakly positive", "strongly positive", or in a more detailed range, or based on a combination of classifications of a plurality of marker molecules.

In one aspect, the long-term hematopoietic stem cells having more undifferentiated cell characteristics of the present disclosure are isolated or enriched using, as a long-term hematopoietic stem cell isolation marker or enrichment marker, one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof.

In addition, in another aspect, the long-term hematopoietic stem cells having more undifferentiated cell characteristics of the present disclosure are isolated using, as a long-term hematopoietic stem cell isolation marker, one or more selected from a combination of CD48(-) and CD34(+), a combination of CD48(-) and c-Kit(+), and a combination of CD48(-),CD34(+), and c-Kit(+). In particular, it is possible to isolate long-term hematopoietic stem cells with extremely high purity using the combination of CD48(-), CD34(+), and c-Kit(+) as a long-term hematopoietic stem cell isolation marker.

In addition, in another aspect, as for the long-term hematopoietic stem cells having more undifferentiated cell characteristics of the present disclosure, long-term hematopoietic stem cells with higher purity can be enriched by combining one or more selected from CD84(+), CD111(+), CD150(-), GPR56(-), Notch-1(-), CD52(-), CD62L(+), CD71(+), CD93(-), CD122(-), CD133(-), CD275(-), CD131(-), CD326(+), CD200(-), CD200R(-), CD324(+), CD40(-), CD11c(-), and CD318(-), in addition to the combination of gene expression patterns.

Preferably, an additionally combined gene expression pattern is selected from CD111(+), CD133(-), CD131(-), CD93(-), CD122(-), CD200(-), and CD324(+). More preferably, an additionally combined gene expression pattern is selected from CD111(+), CD133(-), and CD131(-).

In a case where the marker for isolating or enriching long-term hematopoietic stem cells of the present disclosure is used, differentiated cells may be eliminated from a cell population to be treated in advance. In order to eliminate the differentiated cells, a marker for specifically identifying the differentiated cells, generally called a lineage marker, can be used. For example, a combination of CD11b, CD14, CD19, CD20, CD235ab, CD3, CD4, CD56, and CD8a can be used as the lineage marker, but the lineage marker is not limited thereto.

As a detection target of the marker molecule, various products of genes encoding the marker molecule can be used. In one aspect, a marker molecule expressed on a cell surface is used as a detection target. As another non-limiting example, mRNA transcribed from a gene or a precursor thereof, a peptide or protein translated from the mRNA, or an intermediate product, fragment, or modification thereof, and the like can be used.

A gene product used to identify the hematopoietic stem cells of the present disclosure is not limited to the gene product of the gene specifically described above. In order to further classify the hematopoietic stem cells of the present disclosure, expression or non-expression of one or more additional gene products can be used as an index. In addition, a part of the gene combination can be replaced by other genes. Such a gene can be selected from genes functionally or statistically similar to expression patterns of genes included in the above gene combination. As described below, top additional or alternative genes can be identified by analyzing the gene expression pattern in the hematopoietic stem cells of the present disclosure.

In one aspect, the present disclosure provides a use of a long-term hematopoietic stem cell isolation marker or enrichment marker containing one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof. In one aspect, the present disclosure provides a use of a long-term hematopoietic stem cell isolation marker or enrichment marker containing one or more selected from a combination of CD48(-) and CD34(+), a combination of CD48(-) and c-Kit(+), and a combination of CD48(-),CD34(+), and c-Kit(+).

In addition, in another aspect, long-term hematopoietic stem cells with higher purity can be enriched by combining, as a long-term hematopoietic stem cell isolation marker or enrichment marker, one or more selected from CD84(+), CD111(+), CD150(-), GPR56(-), Notch-1(-), CD52(-), CD62L(+), CD71(+), CD93(-), CD122(-), CD133(-), CD275(-), CD131(-), CD326(+), CD200(-), CD200R(-), CD324(+), CD40(-), CD11c(-), and CD318(-), in addition to the combination of gene expression patterns.

Preferably, an additionally combined gene expression pattern is selected from CD111(+), CD133(-), CD131(-), CD93(-), CD122(-), CD200(-), and CD324(+). More preferably, an additionally combined gene expression pattern is selected from CD111(+), CD133(-), and CD131(-).

### 2. Long-Term Hematopoietic Stem Cells of Present Disclosure

The present disclosure also provides isolated hematopoietic stem cells or a hematopoietic stem cell-containing cell population in which hematopoietic stem cells are enriched having more undifferentiated cell characteristics. In one aspect, such hematopoietic stem cells or cell population is identified by an expression pattern of one, two, three, four, or five or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof. In one aspect, such hematopoietic stem cells or cell population is identified by an expression pattern of one or more selected from a combination of CD48(-) and CD34(+), a combination of CD48(-) and c-Kit(+), and a combination of CD48(-),CD34(+), and c-Kit(+). Typically, the cells or cell population expresses or does not express the molecule as a cell surface protein. As another non-limiting example, the cells or cell population expresses the molecule as mRNA transcribed from a gene or a precursor thereof, a peptide or protein translated from the mRNA, or an intermediate product, fragment, or modification thereof, or the like.

### 3. Method for Producing Isolated or Enriched Long-Term Hematopoietic Stem Cells or Cell Population Containing Long-Term Hematopoietic Stem Cells

The present disclosure provides a method for producing isolated or enriched hematopoietic stem cells or a cell population containing hematopoietic stem cells based on an expression pattern of a marker molecule for isolating or enriching long-term hematopoietic stem cells.

In one example, the method can be performed by operating a cell sorter coupled to a flow cytometer based on, but not limited to, cell surface expression of the marker molecule for identifying long-term hematopoietic stem cells discovered in the present disclosure.

### 4. Method for Evaluating Content and/or Change in Content of Long-Term Hematopoietic Stem Cells

The present disclosure provides a method for evaluating a content and/or a change in content of long-term hematopoietic stem cells in a cell composition containing long-term hematopoietic stem cells using, as an index, the marker molecule for isolating or enriching long-term hematopoietic stem cells of the present disclosure. Typically, a cell population containing hematopoietic stem cells is prepared, an expression level of a marker molecule for isolating or enriching long-term hematopoietic stem cells is measured for cells contained in the cell population, and the expression level is compared with an expression level of a marker molecule in a subject sample or an expression level of a marker molecule at another time point of a cell composition containing the long-term hematopoietic stem cells, thereby evaluating a content and/or a change in content of the long-term hematopoietic stem cells.

The cell population containing hematopoietic stem cells may be cells derived from a living body or cells derived from cells having artificially acquired pluripotency. In addition, the cells for measuring the expression level of the marker molecule may be all or some of cells contained in the cell population.

The expression level of the marker molecule can be measured by a method known to those skilled in the art, and can be measured using, for example, a flow cytometer, an antibody panel, or the like, but is not limited thereto.

### 5. Method for Testing or Evaluating Characteristics of Hematopoietic Stem Cell-Containing Composition

The present disclosure provides a method for testing or evaluating characteristics of a cell composition using, as an index, a marker molecule for isolating or enriching long-term hematopoietic stem cells of the present disclosure in the cell composition containing the long-term hematopoietic stem cells. Typically, a cell population containing hematopoietic stem cells is prepared, an expression level of a marker molecule for isolating or enriching long-term hematopoietic stem cells is measured for cells contained in the cell population, and the expression level is compared with an expression level of a marker molecule in a subject sample or an expression level of a marker molecule in a control sample, thereby testing or evaluating characteristics of the cell composition. The control sample may be a sample whose characteristics as a long-term hematopoietic stem cell-containing cell population are confirmed by an in vivo or in vitro test, or may be a sample at another time point of the cell population to be tested or evaluated.

For example, the cell composition may be a specimen used for hematopoietic stem cell transplantation, for example, cord blood, bone marrow fluid, or a peripheral blood-containing composition, but is not limited thereto. For example, in a cell composition containing long-term hematopoietic stem cells, it is possible to test or evaluate the extent to which the characteristics of the long-term hematopoietic stem cells are changed or not in a cell population before and after culture or before and after storage, using, as an index, an expression level of a marker molecule for isolating or enriching long-term hematopoietic stem cells in a cell composition before and after cell culture and before and after storage for a certain period of time.

In one aspect, the characteristic of the hematopoietic stem cell-containing composition may be quality evaluated according to certain criteria, and for example, a quality such as hematopoietic performance maintenance ability, transplantation compatibility, or storage stability can be evaluated. For example, transplantation compatibility of a test transplantation composition can be tested or evaluated by comparing a test transplantation composition containing long-term hematopoietic stem cells with a standard composition confirmed to be compatible with transplantation using an expression level of a marker molecule for isolating or enriching long-term hematopoietic stem cells as an index.

The cell population containing hematopoietic stem cells may be cells derived from a living body or cells derived from cells having artificially acquired pluripotency, or may be a cell composition obtained by culturing cord blood, bone marrow cells, peripheral blood, or the like, but is not limited thereto. In addition, the cells for measuring the expression level of the marker molecule may be all or some of cells contained in the cell population.

The expression level of the marker molecule can be measured by a method known to those skilled in the art, and can be measured using, for example, a flow cytometer, an antibody panel, or the like, but is not limited thereto.

### 6. Method for Testing Hematopoietic Stem Cell-Containing Composition for Predicting Disease Prognosis or Health Status of Subject

The present disclosure provides a method for testing a hematopoietic stem cell-containing composition for predicting a disease prognosis or a health status of a subject using, as an index, the marker molecule for isolating or enriching long-term hematopoietic stem cells of the present disclosure. Typically, in cells contained in a cell population containing hematopoietic stem cells obtained from a patient or a healthy person, or cells contained in a cell population containing hematopoietic stem cells in a patient or a healthy human body, an expression level of a marker molecule for isolating or enriching long-term hematopoietic stem cells is measured, and the measured expression level is used as an index to predict a disease prognosis or a future health status of a subject. For example, in order to predict a prognosis of a patient or to confirm a therapeutic effect, a cell population containing hematopoietic stem cells is obtained from a patient before and after treatment, and an increase or decrease in proportion of long-term hematopoietic stem cells is tested, and when the proportion of the long-term hematopoietic stem cells is increased or the decrease is suppressed, a preferred prognosis of a patient can be predicted. In another aspect, in a case where a treatment is performed by allotransplantation of a living body-derived hematopoietic stem cell-containing composition, in order to predict a prognosis of a patient, a cell population containing hematopoietic stem cells obtained from a donor, a cell population containing hematopoietic stem cells in a patient before or after transplantation, or a cell population containing hematopoietic stem cells obtained from the patient can also be tested. In another aspect, for cells contained in a cell population containing hematopoietic stem cells obtained from a patient or a healthy person, in order to understand a health condition of the patient or the healthy person, for example, a health condition in a hematopoietic system or to predict a future health status, an expression level of a marker molecule for isolating or enriching long-term hematopoietic stem cells can be tested.

As the cell population containing long-term hematopoietic stem cells, any long-term hematopoietic stem cell-containing cell population such as a bone marrow tissue, cord blood, or peripheral blood can be used. The cells for measuring the expression level of the marker molecule may be all or some of cells contained in the cell population.

The expression level of the marker molecule can be measured by a method known to those skilled in the art, and can be measured using, for example, a flow cytometer, an antibody panel, or the like, but is not limited thereto.

### 7. Method for Modifying Cell Population Containing Long-Term Hematopoietic Stem Cells

The present disclosure provides a method for modifying isolated or enriched hematopoietic stem cells or a cell population containing hematopoietic stem cells that is isolated or enriched by a marker for isolating or enriching long-term hematopoietic stem cells. The modification includes inducing various differentiations of the isolated or enriched hematopoietic stem cells or the cell population containing hematopoietic stem cells. For example, the long-term hematopoietic stem cell-containing cell population can be modified by, but not limited to, culture under specific culture conditions, a treatment by a specific drug or a combination of drugs, genome editing, gene introduction, intracellular introduction of mRNA, antisense oligonucleotides, aptamers, or the like, and a treatment such as fusion with other cells. The culture conditions include, but are not limited to, a composition of a culture medium, a change in the composition of the culture medium, a specific additive contained in the culture medium, a method for adding the additive, a culture temperature or a change mode thereof, a culture humidity or a change mode thereof, an atmospheric pressure or a change mode thereof, a structure or material of a culture vessel or a change mode thereof, a structure or material of a culture scaffold or a change mode thereof, and the like. The drug contains, but is not limited to, an inorganic compound, an organic low molecular weight compound, a high molecular weight compound, a protein, and a nucleic acid. The genome editing includes a method known to those skilled in the art, and includes, for example, a genome editing technique using a CRISPR-Cas system, a TALEN system, or the like, but is not limited thereto. The modification of the long-term hematopoietic stem cell-containing cell population can be confirmed by, but not limited to, a change in type or amount of molecules expressed on the cell surface, a change in type or amount of genes transcribed in the cell, a change in genomic base sequence of the cell, or a change in cell morphology or function associated therewith. For example, in a case where a type or amount of any molecule expressed on the cell surface after modification or gene transcribed in the cell is different from that before modification by 0.1%, 0.5%, 1%, 2%, 3%, 4%, 5%, 6%, 7%, 8%, 9%, or 10% or more, the cells can be modified cells. In addition, the gene introduction includes methods known to those skilled in the art, and includes, but is not limited to, a gene introduction technique using a viral vector, a gene introduction method using an electroporation method, and the like. In addition, fusion with the other cells includes methods known to those skilled in the art, and as the cells to be fused, pluripotent stem cells isolated from a somatic cell population such as human or primate somatic cells, ES cells, induced pluripotent stem cells, or Muse cells by a specific method can be used, but the cells are not limited thereto. The modified cell population can be used as a sample for studying a differentiation mechanism and the like of cells, and can be used for various uses in accordance with its characteristics. In one example, long-term hematopoietic stem cells are isolated or enriched from living tissues of a patient, and the cells are subjected to modifications such as introduction or deletion of genes and adjustment of an expression level by genome editing, gene introduction by a vector, or the like, and then returned to the patient body as functionally modified long-term hematopoietic stem cells, such that the cells can be used for disease treatment and the like. In addition, in another example, long-term hematopoietic stem cells present in a living body are identified by the marker for isolating or enriching long-term hematopoietic stem cells of the present disclosure, and long-term hematopoietic stem cells having an added or modified function by modifications of cells, such as introduction or deletion of genes and adjustment of an expression level in vivo by genome editing or gene introduction, such that it is possible to provide disease treatment, health maintenance, promotion methods, and the like. In one aspect, the modification of these cells may be permanent or temporary. For example, at a specific site in the body, cells may be modified only for a specific period of time.

### 8. Method for Analyzing Cell Population Containing Long-Term Hematopoietic Stem Cells

The present disclosure provides a method for analyzing a long-term hematopoietic stem cell-containing cell population isolated or enriched by a marker for isolating or enriching long-term hematopoietic stem cells to obtain information on physical, chemical, or biological characteristics of the cell population. Physical, chemical, or biological characteristics of isolated or enriched hematopoietic stem cells or hematopoietic stem cells can be evaluated and analyzed using methods known to those skilled in the art. The obtained information can be used in a method for identifying a long-term hematopoietic stem cell-containing cell population based on, for example, physical, chemical, biological characteristics, or a combination thereof.

### 9. Method for Producing Hematopoietic Stem Cells and Classifier for Isolating or Enriching Hematopoietic Stem Cells Based on Physical, Chemical, and Biological Characteristics of Long-Term Hematopoietic Stem Cells

In one example, the physical, chemical, and biological characteristics can be used as a positive control when developing a method for isolating or enriching hematopoietic stem cells. For example, in a case where hematopoietic stem cells are isolated or enriched based solely on the biological characteristics of cells, for example, a proliferative response to a certain chemical substance and the like, physical, chemical, and biological characteristics of cells or a cell population isolated or enriched by the method are compared with physical, chemical, and biological characteristics of hematopoietic stem cells obtained using the marker of the present disclosure, such that a method for isolating or enriching hematopoietic stem cells based on the physical characteristic can be evaluated or characterized. Through such evaluation and characterization, in one aspect, there is provided a method for identifying hematopoietic stem cells using the physical, chemical, and biological characteristics of hematopoietic stem cells obtained using the marker of the present disclosure as indices, and producing a cell population containing hematopoietic stem cells.

### 10. Method for Producing Hematopoietic Stem Cells and Classifier for Isolating or Enriching Hematopoietic Stem Cells Based on Physical, Chemical, and Biological Characteristics of Long-Term Hematopoietic Stem Cells

The physical, chemical, and biological characteristics are used, in one example, to produce hematopoietic stem cells or a classifier for isolating or enriching hematopoietic stem cells. For example, in a case where the classifier is configured by machine learning, physical, chemical, biological characteristics, or a combination or a part thereof obtained from the isolated or enriched hematopoietic stem cells of the present disclosure can be used as teacher data for machine learning.

### 11. Method for Evaluating Physiological Action of Test Substance or Environmental Factor on Hematopoietic Stem Cells Using Long-Term Hematopoietic Stem Cells

The present disclosure provides a method for evaluating a physiological action exerted on a test substance such as a compound or a drug or an environmental factor such as a temperature and a nutritional condition on hematopoietic stem cells using isolated or enriched hematopoietic stem cells or a cell population containing hematopoietic stem cells or various cells obtained by modifying the isolated or enriched hematopoietic stem cells of the cell population containing hematopoietic stem cells.

In the method, the isolated or enriched hematopoietic stem cells or the cell population containing hematopoietic stem cells of the present disclosure or various cells obtained by modifying the isolated or enriched hematopoietic stem cells or the cell population containing hematopoietic stem cells are brought into contact with a test substance such as a compound or a drug by a known method, and a physiological action of an environmental factor such as a temperature or a nutritional condition on the cells, for example, a change in cell function, a change in proliferation state, an effect on survival, and the like are evaluated.

### 12. Method for Evaluating Effect of Culture Conditions on Proliferation of Long-Term Hematopoietic Stem Cells

The present disclosure provides a method for evaluating an effect of culture conditions on proliferation of long-term hematopoietic stem cells using the marker molecule for isolating or enriching long-term hematopoietic stem cells of the present disclosure as an index. The evaluation method of the present disclosure typically includes: preparing a cell population containing hematopoietic stem cells; culturing the cell population containing hematopoietic stem cells under culture conditions to be evaluated; and testing an expression level of the marker molecule for identifying the long-term hematopoietic stem cells of the present disclosure in the cultured cell population, and the expression levels of the marker molecule before and after culture under the culture conditions to be evaluated are compared, or the expression level of the marker molecule after culture under the culture conditions to be evaluated is compared with the expression level of the marker molecule after culture under culture conditions to be as a control. As a result of the comparison, in a case where the proliferation of the long-term hematopoietic stem cells is promoted, the culture conditions to be evaluated are evaluated as culture conditions that promote the proliferation of the long-term hematopoietic stem cells, and when the proliferation of the long-term hematopoietic stem cells is suppressed after changing the culture conditions, the culture conditions to be evaluated are evaluated as culture conditions for suppressing the proliferation of the long-term hematopoietic stem cells.

A method for evaluating whether or not the long-term hematopoietic stem cells are specifically modified is not limited, and those skilled in the art can use a known method. For example, it is evaluated that the long-term hematopoietic stem cells are specifically modified when results such as shortening or extending the time required for cell division of the long-term hematopoietic stem cells identified by the marker molecule for isolating or enriching long-term hematopoietic stem cells of the present disclosure, increasing the number of cells of the long-term hematopoietic stem cells identified by the marker molecule of the present disclosure, and decreasing, maintaining, or increasing a content ratio of the long-term hematopoietic stem cells identified by the marker molecule of the present disclosure in a cell composition containing long-term hematopoietic stem cells are obtained.

The cell population containing hematopoietic stem cells may be cells derived from a living body or cells derived from cells having artificially acquired pluripotency. In addition, the cells for measuring the expression level of the marker molecule may be all or some of cells contained in the cell population.

As factors constituting the culture conditions of the cells, factors usually considered in the art can be evaluated, and for example, the test can be performed by changing the medium component, temperature, humidity, atmospheric configuration, culture substrate, and the like, but is not limited thereto.

### 13. Method for Screening Compound Modifying Characteristics of Long-Term Hematopoietic Stem Cells

The present disclosure provides a method for screening a compound modifying characteristics of long-term hematopoietic stem cells using a marker molecule for isolating or enriching long-term hematopoietic stem cells as an index. In the screening method of the present disclosure, typically, a cell population containing hematopoietic stem cells is prepared, cells or a cell population treated with a candidate compound is compared with untreated cells or cell population, and a compound modifying characteristics of long-term hematopoietic stem cells specified by the marker molecule for isolating or enriching long-term hematopoietic stem cells of the present disclosure is obtained. For example, in a compound that shortens or extends the time required for cell division of long-term hematopoietic stem cells, a compound that increases the number of cells of long-term hematopoietic stem cells, and a cell composition containing long-term hematopoietic stem cells, a compound that decreases, maintains, or increases a content ratio of long-term hematopoietic stem cells, a compound that extends or shortens a survival time of long-term hematopoietic stem cells, a compound that inhibits or promotes cell death of long-term hematopoietic stem cells, a compound that promotes or inhibits modifications (gene introduction, genome editing, drug treatment efficiency, or the like) of long-term hematopoietic stem cells, a compound that differentiates long-term hematopoietic stem cells into specific cell types, a compound that inhibits differentiation into specific cell types, a compound that inhibits or promotes differentiation of long-term hematopoietic stem cells, a compound that inhibits differentiation into specific cell types, and the like can be obtained.

The cell population containing hematopoietic stem cells may be cells derived from a living body or cells derived from cells having artificially acquired pluripotency. In addition, the cells for measuring the expression level of the marker molecule may be all or some of cells contained in the cell population.

### 14. Method for Screening Compound Inducing Long-Term Hematopoietic Stem Cells

The present disclosure provides a method for screening a compound inducing long-term hematopoietic stem cells using a marker molecule for isolating or enriching long-term hematopoietic stem cells as an index. In the screening method of the present disclosure, typically, cells other than long-term hematopoietic stem cells are prepared, the cells or cell population is treated with a candidate compound, and a compound inducing long-term hematopoietic stem cells is obtained using a marker for isolating or enriching long-term hematopoietic stem cells in the treated cells or cell population as an index. The cells other than long-term hematopoietic stem cells are not particularly limited, and for example, cells derived from a living body, and cells having artificially acquired pluripotency, cells modified by gene introduction or the like can be used. For example, cells having artificially acquired pluripotency such as ES cells and iPS cells can be used, but the cells are not limited thereto.

The cells for measuring the expression level of the marker molecule may be all or some of cells contained in the cell population.

### 15. Culture Medium for Modifying Characteristics of Long-Term Hematopoietic Stem Cells

The present disclosure provides a method for producing a culture medium or a culture auxiliary additive that specifically modifies characteristics of long-term hematopoietic stem cells using a marker molecule for isolating or enriching long-term hematopoietic stem cells as an index, and a culture medium or a culture auxiliary additive produced by the method. Typically, a cell population containing hematopoietic stem cells is prepared, the cell population containing hematopoietic stem cells is cultured by a medium having a configuration to be evaluated or a medium containing a culture auxiliary additive to be evaluated, a proliferation state of cells before and after change of culture conditions is evaluated using the marker molecule for isolating or enriching long-term hematopoietic stem cells of the present disclosure as an index, a configuration of the culture medium or the culture auxiliary additive that specifically modifies characteristics of the long-term hematopoietic stem cells is determined, and then a culture medium or a culture auxiliary additive having the configuration is produced. The culture medium and the culture auxiliary additive are configured, for example, as a solid or liquid composition. The culture medium can also have a particular physical structure in a configuration, for example, a shape, a material, a volume, or the like.

A method for evaluating whether or not the characteristics of the long-term hematopoietic stem cells are specifically modified is not limited, and those skilled in the art can use a known method. For example, it is evaluated that the proliferation of the long-term hematopoietic stem cells is promoted when results such as shortening or extending the time required for cell division of the long-term hematopoietic stem cells identified by the marker molecule for isolating or enriching long-term hematopoietic stem cells of the present disclosure, increasing the number of cells of the long-term hematopoietic stem cells identified by the marker molecule of the present disclosure, and decreasing, maintaining, or increasing a content ratio of the long-term hematopoietic stem cells identified by the marker molecule of the present disclosure in a cell composition containing long-term hematopoietic stem cells are obtained.

The cell population containing hematopoietic stem cells may be cells derived from a living body or cells derived from cells having artificially acquired pluripotency. In addition, the cells for measuring the expression level of the marker molecule may be all or some of cells contained in the cell population.

### 16. Culture Method for Promoting Proliferation of Long-Term Hematopoietic Stem Cells

The present disclosure provides a method for adjusting culture conditions for promoting proliferation of long-term hematopoietic stem cells using the marker molecule for isolating or enriching long-term hematopoietic stem cells of the present disclosure as an index. In the adjustment method of the present disclosure, typically, a cell population containing hematopoietic stem cells is prepared, the cell population containing hematopoietic stem cells is cultured under the adjusted culture conditions, a proliferation state of cells before and after change in culture conditions is evaluated using the marker molecule as an index, and then the culture conditions are adjusted so that the proliferation of the long-term hematopoietic stem cells is promoted.

As factors constituting the culture conditions for the cells, factors usually considered in the art can be evaluated. For example, a medium component, a culture auxiliary additive, a temperature, a humidity, an atmospheric configuration, a shape of a culture substrate, a material, a surface treatment, a volume, a shape, and a material of a culture vessel, a surface treatment, a physical stimulus added to the culture vessel, the medium, or the cell itself, a change over time, and the like can be evaluated as factors, but the present disclosure is not limited thereto.

A method for evaluating whether or not proliferation of the long-term hematopoietic stem cells is promoted is not limited, and those skilled in the art can use a known method. For example, it is evaluated that the proliferation of the long-term hematopoietic stem cells is promoted when results such as shortening the time required for cell division of the long-term hematopoietic stem cells identified by the marker molecule for isolating or enriching long-term hematopoietic stem cells of the present disclosure, increasing the number of cells of the long-term hematopoietic stem cells identified by the marker molecule of the present disclosure, and maintaining or increasing a content ratio of the long-term hematopoietic stem cells identified by the marker molecule of the present disclosure in a cell composition containing long-term hematopoietic stem cells are obtained.

The cell population containing hematopoietic stem cells may be cells derived from a living body or cells derived from cells having artificially acquired pluripotency. In addition, the cells for measuring the expression level of the marker molecule may be all or some of cells contained in the cell population.

### 17. Method for Producing Isolated or Enriched Hematopoietic Stem Cells or Cell Population Containing Hematopoietic Stem Cells Using Adjusted Culture Conditions

The present disclosure provides a method for producing isolated or enriched hematopoietic stem cells or a cell population containing hematopoietic stem cells, the method including adjusting culture conditions using the long-term hematopoietic stem cell isolation or enrichment marker molecule of the present disclosure. Typically, a cell population containing hematopoietic stem cells is prepared, and the cell population containing hematopoietic stem cells is cultured under culture conditions adjusted to promote proliferation of long-term hematopoietic stem cells in advance, for example, culture conditions adjusted to promote the proliferation of long-term hematopoietic stem cells by the method for adjusting the culture conditions, thereby producing isolated or enriched hematopoietic stem cells or a cell population containing hematopoietic stem cells.

In another aspect, when the isolated or enriched hematopoietic stem cells or the cell population containing hematopoietic stem cells is cultured, a proliferation state of cells is monitored by the marker molecule for isolating or enriching long-term hematopoietic stem cells of the present disclosure, and culture conditions are adjusted so that proliferation of the long-term hematopoietic stem cells promoted, thereby producing isolated or enriched hematopoietic stem cells or a cell population containing hematopoietic stem cells.

A method for evaluating whether or not proliferation of the long-term hematopoietic stem cells is promoted is not limited, and those skilled in the art can use a known method. For example, it is evaluated that the proliferation of the long-term hematopoietic stem cells is promoted when results such as shortening the time required for cell division of the long-term hematopoietic stem cells identified by the marker molecule for isolating or enriching long-term hematopoietic stem cells of the present disclosure, increasing the number of cells of the long-term hematopoietic stem cells identified by the marker molecule of the present disclosure, and maintaining or increasing a content ratio of the long-term hematopoietic stem cells identified by the marker molecule of the present disclosure in a cell composition containing long-term hematopoietic stem cells are obtained.

The cell population containing hematopoietic stem cells may be cells derived from a living body or cells derived from cells having artificially acquired pluripotency. In addition, the cells for measuring the expression level of the marker molecule may be all or some of cells contained in the cell population.

As factors constituting the culture conditions of the cells, factors usually considered in the art can be evaluated, and for example, the test can be performed by changing the medium component, temperature, humidity, atmospheric configuration, culture substrate, and the like, but is not limited thereto. In one aspect, the culture conditions may be adjusted so as to promote the proliferation of the long-term hematopoietic stem cells from the start of culture of the cell population collected from a living body, and the culture conditions may be adjusted so as to promote the proliferation of the long-term hematopoietic stem cells after the number of cells is increased to a certain extent after culture is performed under conditions that are not optimized for culture of long-term hematopoietic stem cells for a certain period of time.

In another aspect, after culture for a certain period of time, cells can be sorted using a known method such as a cell sorter using the molecular marker for isolating or enriching long-term hematopoietic stem cells of the present disclosure as an index, and culture under adjusted culture conditions can be continued, as necessary. In the adjustment of the culture conditions and the sorting of the cells, a plurality of different methods can be combined, and the same method or different methods can be repeatedly applied.

### 18. Method for Identifying Genetic Modification That Provides Characteristics of Long-Term Hematopoietic Stem Cells to Cells

The present disclosure provides a genetic modification method that provides characteristics of long-term hematopoietic stem cells to cells using the marker for isolating or enriching long-term hematopoietic stem cells as an index. Typically, for some of the cells to be tested, genetically-modified cells modified with candidate genes are prepared, and the marker for isolating or enriching long-term hematopoietic stem cells of the present disclosure in the genetically modified cells is compared with the marker for isolating or enriching long-term hematopoietic stem cells of the present disclosure in cells that are not genetically modified. In addition, in another aspect, the markers for isolating or enriching long-term hematopoietic stem cells of the present disclosure before and after genetic modification in the same cell are compared. As a result of the comparison, a genetic modification that provides a more positive result for the marker for isolating or enriching long-term hematopoietic stem cells of the present disclosure is identified as a genetic modification that provides characteristics of long-term hematopoietic stem cells to cells.

The genetic modification can be performed by various methods known to those skilled in the art, and for example, permanent or transient gene introduction using a nucleic acid or another vector, deletion, addition, mutation, or the like of a gene on a genome or a factor that controls gene expression by genome editing can be used. The mutation includes, but is not limited to, deletion, addition, substitution, or the like of a base in a base sequence constituting a gene or a factor that controls gene expression.

The genetically modified cells may be cells derived from a living body or cells derived from cells having artificially acquired pluripotency. In addition, the cells for testing the marker may be all or some of cells contained in the cell population.

### 19. Composition or Kit for Identifying, Isolating, or Enriching Long-Term Hematopoietic Stem Cells

The present disclosure provides a composition or kit for identifying, isolating, or enriching long-term hematopoietic stem cells, the composition or kit containing a reagent for quantifying or detecting a marker molecule for identifying, isolating, or enriching long-term hematopoietic stem cells. As the reagent for quantifying or detecting a marker molecule, a reagent usually used in the art can be used, and for example, an antibody, a nucleic acid, a low molecular weight compound, a high molecular weight compound, or the like labeled with a fluorescent dye can be used, but the reagent is not limited thereto. The composition or kit for identifying, isolating, or enriching long-term hematopoietic stem cells of the present disclosure includes a composition or kit for identifying long-term hematopoietic stem cells in a living body in addition to a composition or kit that can be used to identify, isolate, or enrich long-term hematopoietic stem cells in a cell population obtained from a living body. The composition or kit for identifying long-term hematopoietic stem cells can further contain a drug or the like for modifying the function of the long-term hematopoietic stem cells, and provides a therapeutic agent or a treatment method for a disease to be treated by modifying the function of long-term hematopoietic stem cells.

### 20. Drug Containing Long-Term Hematopoietic Stem Cells and Cells Obtained by Modifying Long-Term Hematopoietic Stem Cells

The present disclosure provides a drug for treating a disease by being administered to a patient, the drug containing long-term hematopoietic stem cells isolated or enriched by a marker for isolating or enriching long-term hematopoietic stem cells, or various cells obtained by modifying the long-term hematopoietic stem cells, a drug used for pre-emptive medical care for a potential disease, a drug used for preventive medical care or health maintenance (improvement) for a healthy person, or a treatment or therapy using the drug. In a case where the long-term hematopoietic stem cells of the present disclosure or various cells obtained by modifying the long-term hematopoietic stem cells are used as a drug, long-term hematopoietic stem cells obtained from a cell population derived from a patient or various cells obtained by modifying the long-term hematopoietic stem cells may be administered to the patient, or long-term hematopoietic stem cells obtained from a cell population derived from another donor or various cells obtained by modifying the long-term hematopoietic stem cells may be administered to the patient. In addition, another drug or treatment method can be used in combination. For example, from a patient or a donor, long-term hematopoietic stem cells isolated or enriched by a marker for isolating or enriching long-term hematopoietic stem cells of the present disclosure, or from a hematopoietic stem cell-containing cell population derived from induced pluripotent cells generated from somatic cells obtained from a patient or a donor, long-term hematopoietic stem cells isolated or enriched by a marker for isolating or enriching long-term hematopoietic stem cells of the present disclosure can be administered to a patient having a hematopoietic disorder as a drug for treating the disorder. As another example, long-term hematopoietic stem cells can be isolated or enriched by the marker for isolating or enriching long-term hematopoietic stem cells of the present disclosure obtained from a hematopoietic stem cell-containing cell population obtained from biological tissues of a patient with or without an artificial pluripotent process, the long-term hematopoietic stem cells can be subjected to modifications such as introduction of a gene useful for therapy and genome editing, and then the modified long-term hematopoietic stem cells can be administered to a patient as a drug for treating a disease.

The cell population containing hematopoietic stem cells may be cells derived from a living body or cells derived from cells having artificially acquired pluripotency. In addition, the cells for measuring the expression level of the marker molecule may be all or some of cells contained in the cell population.

Examples of the disease or disorder ameliorated or prevented by administering the long-term hematopoietic stem cells include, but are not limited to, a chronic granulomatous disease (CGD), adrenoleukodystrophy (ALD), metachromatic leukodystrophy (MLD), Gaucher's disease, Fabry disease, mucopolysaccharidosis, HIV, ADA-SCID, X-SCID, and thalassemia.

The genetically modified cells can be produced by various methods known to those skilled in the art, and for example, permanent or transient gene introduction using a nucleic acid or another vector, deletion, addition, mutation, or the like of a gene on a genome or a factor that controls gene expression by genome editing can be used. The mutation includes, but is not limited to, deletion, addition, substitution, or the like of a base in a base sequence constituting a gene or a factor that controls gene expression.

The genetically modified cells may be cells derived from a living body or cells derived from cells having artificially acquired pluripotency. In addition, the cells for testing the marker may be all or some of cells contained in the cell population.

### Examples

Hereinafter, the present disclosure will be described in more detail using Examples, but these Examples are examples for description, and the present disclosure is not limited to these Examples.

### Methods and Materials

### (1) Cell Source

Fresh cord blood (provided by Kobe City Medical Center General Hospital and Adachi Hospital (Kobe-shi)) was used as cord blood. As bone marrow fluid, frozen cells, human bone marrow CD34+ progenitor cells, 2 M-101A (Lonza Group AG, Basel, Switzerland) were used. As peripheral blood, frozen cells, mobilized human peripheral blood CD34+ hematopoietic stem cells, positive selection, and 4Y-101C (Lonza Group AG, Basel, Switzerland) were used.

### (2) Collection of Nucleated cells (Removal of Red Blood Cells)

Removal and enrichment treatment of red blood cells were performed using EasySep RBC Depletion Reagent (manufactured by STEMCELL Technologies) using the cell source obtained as described above to obtain a cell population containing hematopoietic stem cells.

### (3) Enrichment of CD34-Positive Cells

The cell population containing hematopoietic stem cells obtained above was crudely purified using EASYSep Human Cord Blood CD34 Positive Selection Kit II (manufactured by STEMCELL Technologies) to obtain a CD34-positive cell fraction.

### (4) Cell Staining

The following antibodies were added to the cell population (CD34-positive enriched cell fraction) obtained by enriching the hematopoietic stem cells and progenitor cells obtained above, and cell staining was performed. The cell staining with antibodies was performed by incubation on ice for 30 minutes. Antibodies contained in CD11b, CD14, CD19, CD20, CD235ab, CD3, CD4, CD56, CD8a, CD34, CD38, CD90, CD45RA, CD49f, and LEGENDScreen Human PE Kit (manufactured by BioLegend).

### (5) Cell Analysis and Cell Sorting

The sample after cell staining was filtered using FACS tube with a cell strainer (manufactured by FALCON), and then dead cell staining was performed. In the dead cell staining, Propidium Iodide Solution (manufactured by BioLegend), SYTOX-Red (manufactured by ThermoFisher SCIENTIFIC Inc.), and the like were used. In analysis and sorting of cells, FACSAria II or III (manufactured by BD) was used. The collected data was analyzed using FlowJo (manufactured by BD) or an algorithm developed by the applicant (also disclosed in PCT/JP2019/051270, PCT/JP2020/025915, and the like). Note that the algorithm is an algorithm that identifies a direction of differentiation in a cell arrangement spatial structure by generating a cell arrangement spatial structure based on the similarity of a plurality of numerical data selected from numerical data on quantitative physical characteristics and/or numerical data on biological characteristics obtained by the cell staining and the like, generating a cell arrangement spatial structure in which cells located close to each other on a differentiation lineage occupy positions close to each other, and using a known differentiation marker and the like.

### (6) CFU Assay

From the analysis result of the cell staining, a candidate fraction likely to contain long-term hematopoietic stem cells was identified, and cells were sorted one by one in a 96-well plate in which 25 ul of MethoCult H4435 Enriched (manufactured by STEMCELL Technologies) was seeded in each well. 12.5 ul of Iscove's MDM with 2% FBS (manufactured by STEMCELL Technologies) was added to the wells subjected to the cell sorting. 200 ul of sterilized water was added to the outermost well of the plate so as to prevent drying. Thereafter, the cells were cultured in a CO₂ incubator under conditions of 37°C and 5%CO2 for 2 weeks, the colonies formed were imaged with an optical microscope (manufactured by KEYENCE Corporation), the number of colonies was visually counted, and the colony morphology was visually discriminated. Colony Forming Unit-Granulocyte, Erythroid, Macrophage, and Megakaryocyte (CFU-GEMM) derived from myeloid progenitor cells, erythroblast burst-forming cells Burst Forming Unit-Erythroid (BFU-E), and Granulocyte-macrophage colony-forming cells Colony Forming Unit-Granulocyte and Macrophage (CFU-GM) were discriminated and counted from the colony morphology.

### (7) Experiment of Transplantation into Immunodeficient Mice

Fresh or cultured cells were transplanted into immunodeficient mice by the following method, and the hematopoietic ability was evaluated. The cell fraction sorted by BD FACSAriaIII was administered into the tail vein or bone marrow of NOG mice (NOD/Shi-scid, IL-2RyKO Jic) irradiated with 2.5 Gy. After transplantation, peripheral blood or bone marrow cells were collected every 4 weeks, red blood cells were disrupted using Lysing Buffer (manufactured by BD) as necessary, and then donor-derived chimerism was measured with FACS Canto. Antibodies against the following were used for cell staining: human CD56, human CD3, human CD16, human CD19, human CD14, human CD45, mouse Gr-1, and mouse CD45, and Propidium Iodide Solution (manufactured by BioLegend) was used for dead cell staining.

### (8) Cell Culture

On U-bottom 96-well plate (manufactured by CORNING Corporation) to which 200 ul of IMDM (manufactured by GIBCO), 1% Insulin-Transferrin-Selenium_Ethanolamine (ITS-X) (manufactured by Gibco), 1% Penicillin-Streeptomycin-Glutamin) (manufactured by Gibco), 50 ng/ml of human Stem Cell Factor (manufactured by PeproTech, Inc.), and human Thrombopoietin (manufactured by PeproTech, Inc.) were added, the cells were sorted using FACSAriaIII so that a candidate cell fraction was 300 cells/well. The sorted cells were cultured in a CO₂ incubator under conditions of 37°C and 5%CO₂ for one week. After culture, the number of viable cells, CD34+CD117+fraction number, CD34+CD117+CD48-fraction number, CD34+CD117+CD48+number, the rate of viable cells, CD34+CD117+fraction number, CD34+CD117+CD48-fraction rate, CD34+CD117+CD48+rate, and the like were analyzed using CD11b, CD14, CD19, CD20, CD235ab, CD3, CD4, CD56, CD8a, CD34, CD38, CD117, and CD48 as markers and using FlowJo (BD). CD34+CD117+CD48-fraction is a fraction mainly composed of long-term hematopoietic stem cells.

### (9) Gene Introduction

An infection experiment was performed using a lentivirus Lenti-LabelerTM virus (manufactured by Funakoshi Co., Ltd.) containing a reporter gene. A virus fluid having 1x10e8IFU/ml of a titer was added to a 96-well plate (manufactured by CORNING Corporation) coated with retronectin (manufactured by Clontech Laboratories, Inc.) to prepare a plate coated with virus. 1,000 or more cells from the candidate cell fraction were sorted and seeded in each well. The cells were infected in 200 ul of IMDM/5ng/ml SCF/5ng/ml TPO/1% Pen/St/Glu for 24 hours. The infected cells were then subjected to cell culture, a CFU assay, a transplantation experiment, and the like.

### Example 1: Generation of Cell Arrangement Spatial Structure in Which Cells Located Close to Each Other on Differentiation Lineage Occupy Positions Close to Each Other

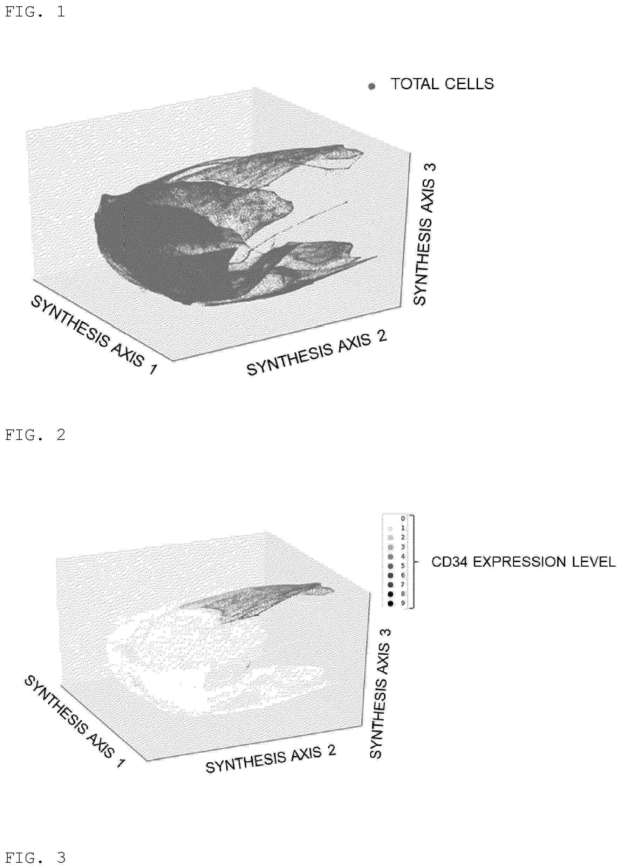

Removal and enrichment treatment of red blood cells were performed on the fresh cord blood obtained from a living body to obtain a cell population containing hematopoietic stem cells. The cell population containing hematopoietic stem cells was crudely purified using EASYSep Human Cord Blood CD34 Positive Selection Kit II (manufactured by STEMCELL Technologies) to obtain a CD34-positive cell fraction. The CD34-positive cell fraction was subjected to dead cell staining using antibodies contained in CD11b, CD14, CD19, CD20, CD235ab, CD3, CD4, CD56, CD8a, CD34, CD38, CD90, CD45RA, CD49f, and LEGENDScreen Human PE Kit (manufactured by BioLegend), and the staining intensity of each antibody was quantified for each cell. A cell arrangement spatial structure in which cells located close to each other on a differentiation lineage occupied positions close to each other was generated using the quantified data. A part of the structure is visualized and illustrated in Figs. 1 and 2. Fig. 1 illustrates, as a two-dimensional image, the position of living cells assigned to a three-dimensional space including a synthesis axis 1, a synthesis axis 2, and a synthesis axis 3 generated from a combination of the quantification data after dead cell staining. Fig. 2 visually illustrates an expression level of CD34, which is known to show a characteristic expression pattern in hematopoietic stem cells, for each cell assigned to a position to the spatial structure. The CD34-positive cells are unevenly distributed in a part of the spatial structure.

### Example 2: Relationship Between Long-Term Hematopoietic Stem Cells and Expression Pattern of CD34

In order to examine a relationship between the long-term hematopoietic stem cells and the expression pattern of CD34, CD3(-)CD34(-) (CD3-negative and CD34-negative) cells or CD3(-)CD34(+) (CD3-negative and CD34-positive) cells separated from human cord blood were transplanted into immunodeficient mice, and the chimerism after transplantation was tested. When the chimera rate was tested at 4 weeks, 8 weeks, and 12 weeks after transplantation, CD3(-)CD34(+) cells showed significantly higher survival rate and chimera rate than CD3(-)CD34(-) cells. The results are illustrated in Fig. 3. Note that the negative and positive fractions of each marker were according to the cell sorting result of BD FACSAriaIII used. The present result shows that the expression pattern of CD34(+) is associated with the long-term hematopoietic stem cells.

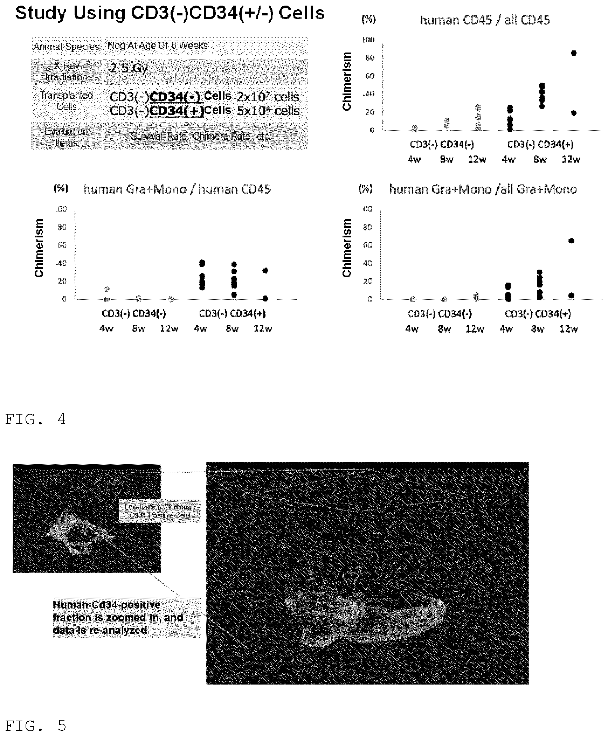

In order to specifically analyze the cell arrangement in the CD34(+) fraction in the cell arrangement spatial structure in which cells located close to each other on a differentiation lineage occupied positions close to each other, the cells in the CD34(+) fraction were arranged in a space generated using the synthetic axis suitable for unfolding the CD34(+) fraction. One example is illustrated in Fig. 4.

Conventionally, it has been reported that the human long-term hematopoietic stem cells were present in the fraction of CD38(-), and as a marker set for identifying human long-term hematopoietic stem cells, a marker set containing CD34(+), CD38(-), CD45RA(-), and CD90(+) and the like have been proposed (Science, 2011, Vol. 333, pp. 218-221, Cell Stem Cell, 2007, Vol. 1, pp. 635-645).

Therefore, the undifferentiation of the CD38(+) and CD38(-) fractions in the CD34(+) fraction were confirmed again. Specifically, the CFU assay was performed on the fraction P6 (CD34(+) and CD38(+)), which was CD38(+), the fraction P7 (CD34(+), CD38(-), CD45RA(-), and CD90(+)), the fraction P8 (CD34(+), CD38(-), CD45RA(-), and CD90(-)), and the fraction P9 (CD34(+), CD38(-), CD45RA(+), and CD90(-)) related to the conventionally proposed CD38(-) fraction, and each fraction was tested for hematopoietic stem cell proliferation/differentiation potency. As a result, P7 and P9 fractionated by the conventionally proposed CD38(-) marker showed extremely low hematopoietic stem cell proliferation/differentiation potency (Fig. 5). On the other hand, since the cells in the fraction P6, which was CD38(+), also showed high hematopoietic stem cell proliferation/differentiation potency, it was understood that the long-term hematopoietic stem cells were distributed not only in the conventionally reported CD34(+) and CD38(-) fractions but also in the entire CD34(+) fraction. Therefore, there was a need to search for a novel marker capable of isolating and enriching the long-term hematopoietic stem cells present in the CD34(+) fraction.

### Example 3: Cell Function Analysis at a single cell level

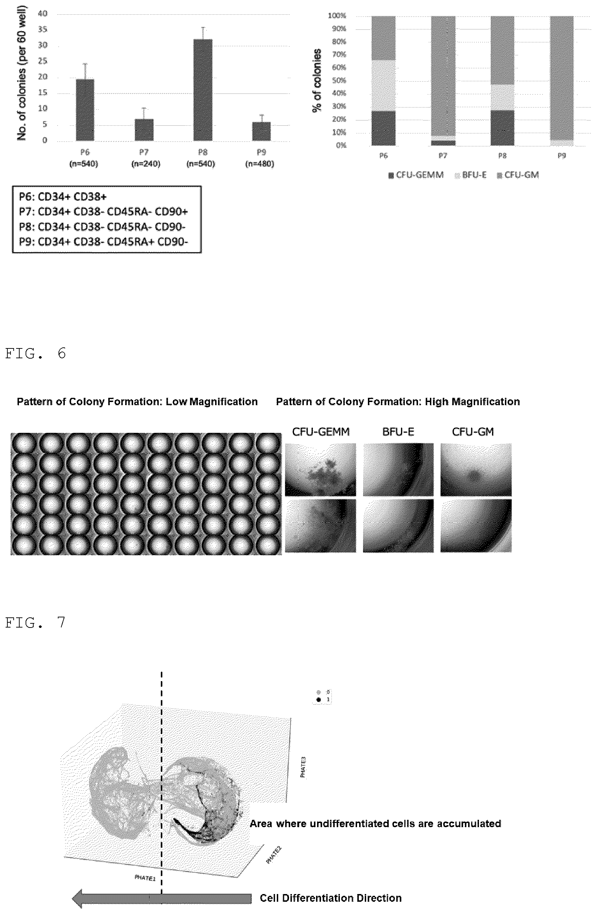

In order to confirm undifferentiation of cells identified by a marker for identifying human long-term hematopoietic stem cells, a CFU assay was performed at a single cell level. One example is illustrated in Fig. 6. By combining the cell function analysis result at the a single cell level with the index sorting analysis, it is possible to associate the undifferentiation of each cell with the expression pattern of each marker. A cell differentiation direction in the cell arrangement spatial structure was determined by arranging the relationship between the marker and the undifferentiation obtained by the analysis on the cell arrangement spatial structure in which cells located close to each other on a differentiation lineage occupied positions close to each other. Fig. 7 illustrates an example of a result of performing the analysis on the CD34-positive cells.

### Example 4: Marker for Enriching or Isolating Long-Term Hematopoietic Stem Cells

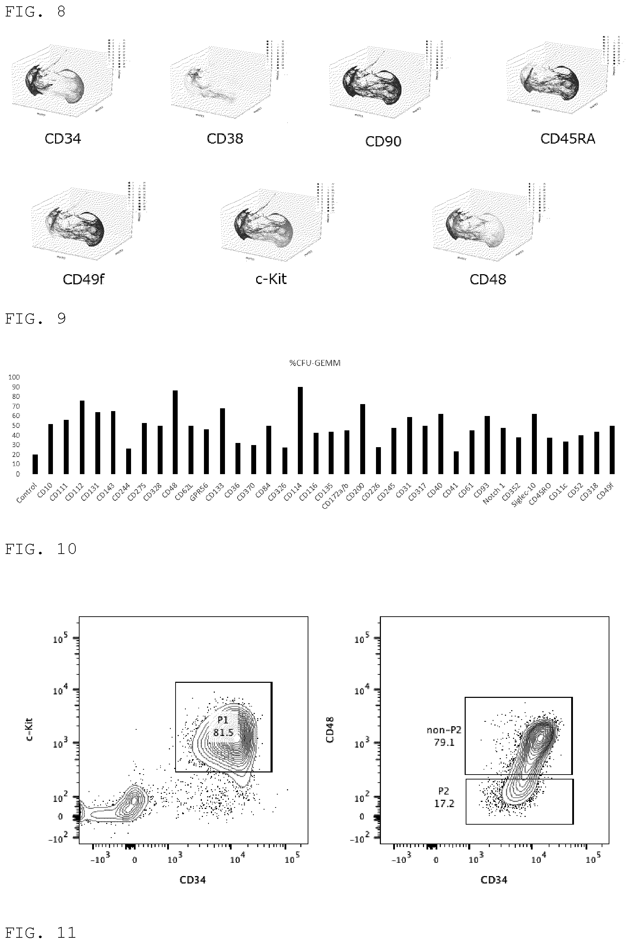

For the CD34-positive cells, a molecular marker in which expression was unevenly distributed in the differentiation direction determined in the cell arrangement spatial structure in which cells located close to each other on a differentiation lineage occupied positions close to each other was identified. One example is illustrated in Fig. 8. A marker molecule expressed more unevenly in the undifferentiation direction and a marker molecule expressed more unevenly in the differentiation direction were identified.

When the proliferation/differentiation potency of the hematopoietic stem cells of the cells identified by these markers were confirmed by a CFU assay, the cells identified by these markers showed higher proliferation/differentiation potency of the hematopoietic stem cells than the control (CD34(+) cells) (Fig. 9).

These markers included the following:
CD10, CD111, CD112, CD131, CD143, CD18, CD244, CD275, CD328, CD48, CD62L, GPR56, CD133, CD36, CD370, CD84, CD326, CD105, CD114, CD116, CD135, CD172a/b, CD200, CD226, CD245, CD31, CD317, CD40, CD41, CD61, CD93, Notch1, CD352, Siglec-10, CD45RO, CD11c, CD52, CD92, CD318, CD49f, c-Kit, and CD34.

Note that as confirmed in Example 2, since there are almost no long-term hematopoietic stem cells in the CD34(-) fraction, the marker for enriching long-term hematopoietic stem cells in a cell population pre-enriched with CD34(+) can be used alone as a marker for enriching long-term hematopoietic stem cells even when applied to a cell population not pre-enriched with CD34(+).

### Example 5: Marker for Identifying Long-Term Hematopoietic Stem Cells in More Detail

Among the identified markers, the characteristics of the cells identified by a marker containing a combination of c-Kit(+), CD34(+), and CD48(-) were analyzed in more detail.

An example of a FACS plot when a cell population pre-enriched with CD34(+) from cord blood is gated on c-Kit(+) and CD34(+) (fraction P1) and is further gated on CD48(-) (fraction P2) is illustrated in Fig. 10. Most of cells in the fraction P1 were fractionated into CD48(+) (fraction non-P2), and only some of cells were fractionated into P2.

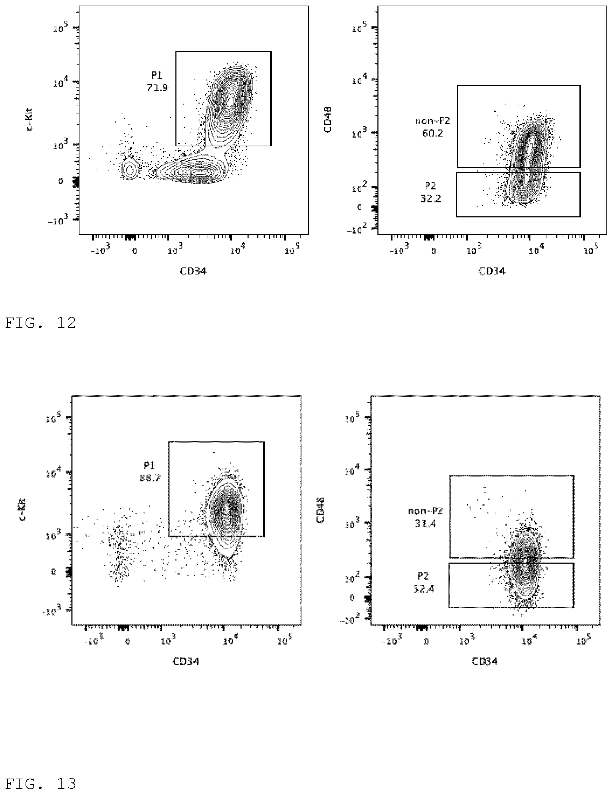

In addition, when similar fractionation was performed using a bone marrow cell sample, a fractionation pattern extremely similar to that of the cord blood sample was shown (Fig. 11). The mobilized peripheral blood sample also showed a significantly similar fraction pattern to that of the cord blood sample (Fig. 12).

The results of comparing the number of cells in the CD34-positive fraction and the P2 fraction contained in 1 ml of cord blood are illustrated in Fig. 12. It is confirmed that the cells fractionated in the P2 fraction is a small fraction of the cell population pre-enriched with CD34(+). Note that since most of the cord blood is CD34(-), a proportion of the cells in the P2 fraction in the entire cord blood sample is extremely low.

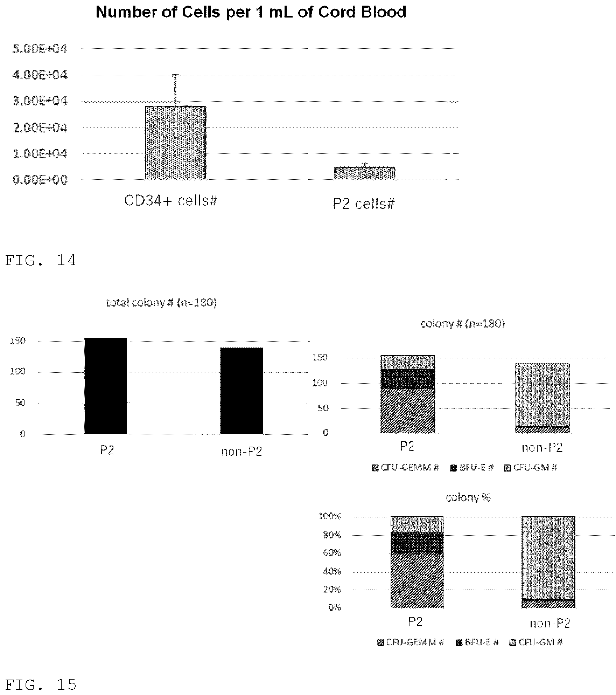

The proliferation/differentiation potency of the hematopoietic stem cells in the cell fraction (P2) identified by c-Kit(+), CD34(+), and CD48(-) and the cell fraction (non-P2) identified by c-Kit(+), CD34(+), and CD48(+) were compared by a CFU assay. As a result, it was confirmed that the P2 fraction had extremely high proliferation/differentiation potency of the hematopoietic stem cells (Fig. 14).

Undifferentiation of the cell fraction P2 and the cell fraction non-P2 were compared by a transplantation test. The cell transplantation was performed at 12 hours or 24 hours after irradiation, and a chimerism in human blood cells (human CD45-positive cells) and a chimerism in human granulocyte cells (hGra) in bone marrow at 20 weeks after transplantation were tested. As a result, it was confirmed that all the results showed an extremely high chimera rate of the cell fraction P2 (Fig. 15).

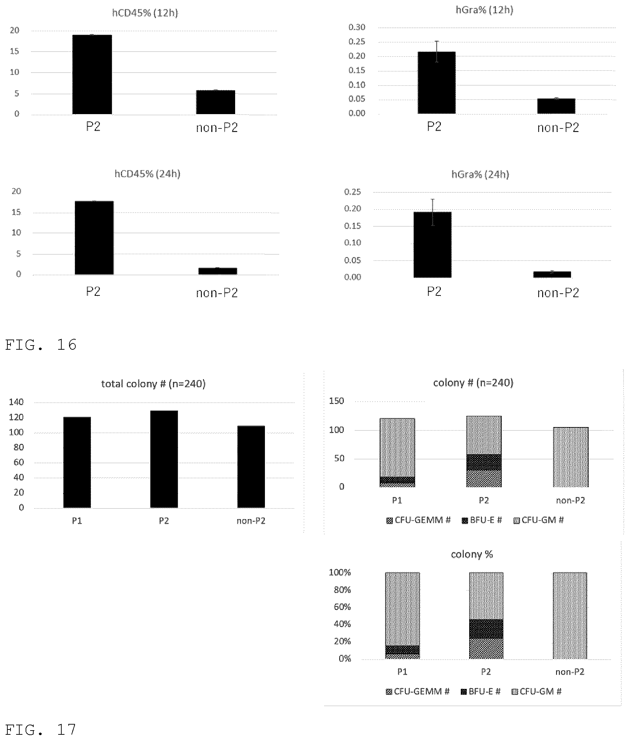

A cell fraction (P2) identified by c-Kit(+), CD34(+), and CD48(-) and a cell fraction (non-P2) identified by c-Kit(+), CD34(+), and CD48(+) were obtained as described above using a bone marrow cell sample and a mobilized peripheral blood sample, and proliferation/differentiation potency of hematopoietic stem cells were compared by a CFU assay. As a result, it was confirmed that the P2 fraction had extremely high proliferation/differentiation potency of the hematopoietic stem cells. The result of the bone marrow cell sample is illustrated in Fig. 16 and the result of the mobilized peripheral blood sample is illustrated in Fig. 17. The result of the P1 fraction, which is a fraction identified by c-Kit(+) and CD34(+), is also illustrated.

The present result shows that long-term hematopoietic stem cells can be extremely efficiently enriched or isolated using a marker containing a combination of c-Kit(+), CD34(+), and CD48(-). In addition, as illustrated in the left plots of Figs. 10 and 11, most of the cells in the CD34(+) fraction were c-Kit(+). In addition, as illustrated in Fig. 5, since the long-term hematopoietic stem cells are selectively fractionated into a fraction of CD34(+), it is understood that the marker of c-Kit(+) can enrich the long-term hematopoietic stem cells similarly to the marker of CD34 (+) .

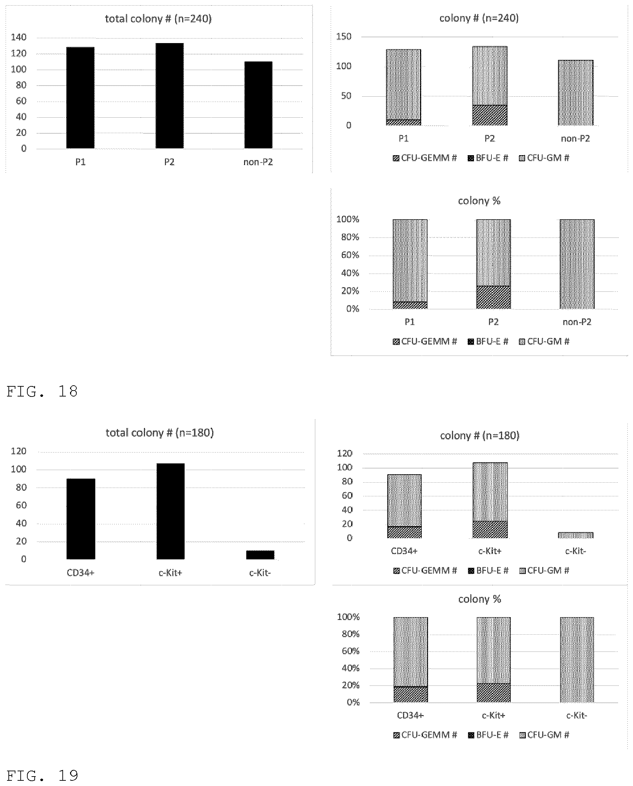

In fact, when the CD34(+) fraction was fractionated into the c-Kit(+) fraction and the c-Kit(-) fraction for the cord blood sample, and the proliferation/differentiation potency of the hematopoietic stem cells were compared by the CFU assay for each fraction, the c-Kit (+) fraction showed almost the same proliferation/differentiation potency of the hematopoietic stem cells as that of the CD34(+) fraction, whereas the c-Kit(-) fraction showed almost no proliferation/differentiation potency of the hematopoietic stem cells (Fig. 18). Therefore, it is understood that a marker containing a combination of CD48(-) and CD34(+) or a combination of CD48(-) and c-Kit(+) can also be used as a marker capable of efficiently enriching or isolating long-term hematopoietic stem cells.

### Example 6: Additional Markers

Additional markers for fractionating the P2 fraction in more detail were identified by analysis using the cell arrangement spatial structure in which located close to each other on a differentiation lineage occupied positions close to each other. Specifically, a marker unevenly distributed in the differentiation direction or undifferentiation direction in the above analysis was set as a candidate, cells in the P2 fraction were divided into two, for example, a test fraction and a control fraction based on the expression level of the marker, and the proliferation/differentiation potency of the hematopoietic stem cells in the fractions were compared by a CFU assay.

From the obtained results, the efficiency of enrichment of the long-term hematopoietic stem cells in the test fraction was quantified by the following equation.

Enrichment ratio = proportion of CFU-GEMM in colonies generated from test fraction/proportion of CFU-GEMM in colonies generated from control fraction

When the proportions occupied by CFU-GEMMs in the colonies generated from the test fraction and the control fraction are the same as each other, the enrichment ratio is 1.

The analysis results are illustrated in Fig. 19. Note that (+) and (-) of each marker are indicated as (+) indicating a relatively high expression level and (-) indicating a relatively low expression level when P2 is divided into two, and have different meanings from negative and positive presented by the cell sorter.

These additional markers include:
CD84(+), CD111(+), CD150(-), GPR56(-), Notch-1(-), CD52(-), CD62L(+), CD71(+), CD93(-), CD122(-), CD133(-), CD275(-), CD131(-), CD326(+), CD200(-), CD200R(-), CD324(+), CD40(-), CD11c(-), and CD318(-).

### Example 7: Identification of Long-Term Hematopoietic Stem Cells by Marker After In Vitro Culture

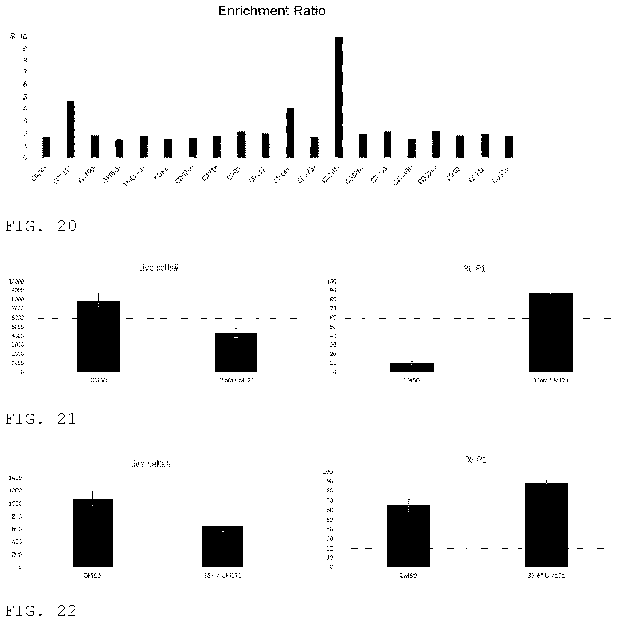

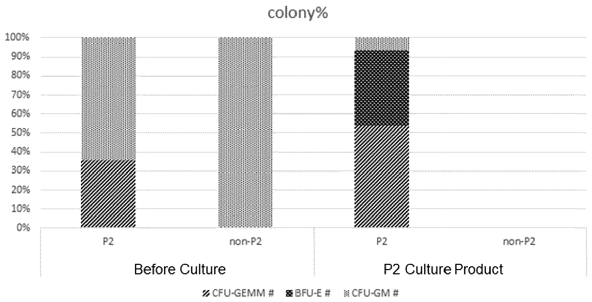

A test was carried out to confirm whether the marker for isolating or enriching long-term hematopoietic stem cells of the present disclosure could identify long-term hematopoietic stem cells after an in vitro culture. For the cell population cultured using a compound UM171 known to promote the proliferation of hematopoietic stem cells, the proportion of cell identified by a marker set of c-Kit(+), CD34(+), and CD48(-) was tested. 300 cells contained in the P2 fraction or the non-P2 fraction were cultured under a condition of addition or non-addition of UM171, and a total cell number and a ratio of undifferentiated cells (P1) after 7 days were tested. The result of the P2 fraction is illustrated in Fig. 20, and the result of the non-P2 fraction is illustrated in Fig. 21. It is understood from the present test that the marker for isolating or enriching long-term hematopoietic stem cells of the present disclosure can identify long-term hematopoietic stem cells after in vitro culture. The fact that the marker for isolating or enriching long-term hematopoietic stem cells of the present disclosure can identify long-term hematopoietic stem cells after in vitro culture can be further confirmed by a functional test such as a CFU assay. After cells in the P2 fraction
fractionated from the cord blood sample with the marker set of c-Kit(+), CD34(+), and CD48(-) were cultured for 7 days, the cell population after culture was fractionated into the P2 fraction (after culture) and the non-P2 fraction (after culture) using the marker set of c-Kit(+), CD34(+), and CD48(-), and a CFU assay was performed for each fraction. As a result, all cells in the cell population after culture were fractionated into the P2 fraction (after culture), and no cells fractionated into the non-P2 fraction (after culture) were confirmed. When the CFU assay was performed on the P2 fraction (after culture), a high content ratio of undifferentiated cells (CFU-GEMM cells) was confirmed from the P2 fraction (after culture) as with the P2 fraction before culture (Fig. 22).

It is shown from these results that the marker for isolating or enriching long-term hematopoietic stem cells of the present disclosure can identify long-term hematopoietic stem cells after the in vitro culture.

### Example 8: Gene Introduction into Isolated or Enriched Long-Term Hematopoietic Stem Cells

In order to confirm whether the long-term hematopoietic stem cells isolated or enriched by the marker for isolating or enriching long-term hematopoietic stem cells of the present disclosure maintain the characteristics of the long-term hematopoietic stem cells after gene introduction, a functional test by a CFU assay is performed. Specifically, a GFP reporter gene is introduced into the cells contained in the P2 fraction, the non-P2 fraction, or the DC34-positive fraction with a lentiviral vector, and a CFU assay is performed on these gene-introduced cells to confirm that the cells into which the gene derived from the P2 fraction is introduced maintain proliferation/differentiation potency.

### Industrial Applicability

The present disclosure provides long-term hematopoietic stem cells, a method for isolating or enriching long-term hematopoietic stem cells, a method for producing isolated or enriched long-term hematopoietic stem cells or a cell population containing long-term hematopoietic stem cells, a method for evaluating isolated or enriched long-term hematopoietic stem cells or a cell population containing long-term hematopoietic stem cells, a composition or kit for detecting, isolating, or enriching isolated or enriched long-term hematopoietic stem cells or a cell population containing long-term hematopoietic stem cells, a method for screening a compound modifying characteristics of long-term hematopoietic stem cells, and a method for evaluating and adjusting culture conditions, and contributes to development of a drug using long-term hematopoietic stem cells.

## Claims

1. A hematopoietic stem cell isolation marker or enrichment marker comprising one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof.

2. The hematopoietic stem cell isolation marker or enrichment marker according to claim 1, wherein the hematopoietic stem cell isolation marker or enrichment marker contains one or more selected from a combination of CD48 and CD34, a combination of CD48 and c-Kit, and a combination of CD48, CD34, and c-Kit.

3. Isolated hematopoietic stem cells or a hematopoietic stem cell-containing cell population in which hematopoietic stem cells are enriched that is identified by an expression pattern of one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof.

4. The isolated hematopoietic stem cells or the hematopoietic stem cell-containing cell population in which hematopoietic stem cells are enriched according to claim 3, wherein the isolated hematopoietic stem cells or the hematopoietic stem cell-containing cell population in which hematopoietic stem cells are enriched is identified by an expression pattern of one or more selected from a combination of CD48(-) and CD34(+), a combination of CD48(-) and c-Kit(+), and a combination of CD48(-), CD34(+), and c-Kit(+).

5. A method for producing isolated or enriched hematopoietic stem cells or a cell population containing hematopoietic stem cells, the method comprising:
preparing a cell population containing hematopoietic stem cells; and
isolating or enriching cells using, as an index, an expression pattern of one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof.

6. The method for producing isolated or enriched hematopoietic stem cells or a cell population containing hematopoietic stem cells according to claim 5, wherein
an expression pattern of one or more selected from a combination of CD48(-) and CD34(+), a combination of CD48(-) and c-Kit(+), and a combination of CD48(-), CD34(+), and c-Kit(+) is used as an index.

7. Hematopoietic stem cells identified, isolated, or enriched by the marker according to claim 1 or 2, the isolated or enriched hematopoietic stem cells or the cell population containing hematopoietic stem cells according to claim 3 or 4, or isolated or enriched hematopoietic stem cells or a cell population containing hematopoietic stem cells produced by the method according to claim 5 or 6, the hematopoietic stem cells or the cell population containing hematopoietic stem cells further comprising a modification.

8. A method for evaluating a content and/or a change in content of hematopoietic stem cells in the cell population, the method comprising:
preparing a cell population containing hematopoietic stem cells; and
measuring, in each cell, an expression level of a marker containing one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48 of each cell, or one or more selected from a combination of CD48 and CD34, a combination of CD48 and c-Kit, and a combination of CD48, CD34, and c-Kit, or a combination thereof.

9. A test method for a cell population containing hematopoietic stem cells, the test method comprising:
preparing a cell population containing hematopoietic stem cells; and
testing quality of the cell population using, as an index, an expression level of a marker containing one or more selected from CD10, CD111, CD112, CD131, CD143, CD244, CD275, CD328, CD48, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in the cells contained in the cell population containing hematopoietic stem cells.

10. The test method according to claim 9, wherein the quality is hematopoietic capacity maintenance ability, transplantation compatibility, or storage stability.

11. A test method for a cell population containing hematopoietic stem cells, the test method comprising:
obtaining an expression level of a marker containing one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in cells contained in a cell population containing hematopoietic stem cells derived from a subject; and
comparing the expression level of the marker with a control for predicting a disease prognosis or a health status of the subject.

12. A method for screening a compound modifying characteristics of hematopoietic stem cells, the method comprising:
preparing a cell population containing hematopoietic stem cells;
treating the cell population containing hematopoietic stem cells with a candidate compound; and
selecting a candidate compound using, as an index, an expression level of a marker containing one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in the cells treated with the candidate compound.

13. A method for adjusting culture conditions to modify characteristics of hematopoietic stem cells, the method comprising:
preparing a cell population containing hematopoietic stem cells;
culturing the cell population containing hematopoietic stem cells under adjusted culture conditions; and
determining culture conditions using, as an index, an expression level of a marker containing one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in the cells cultured under the adjusted culture conditions.

14. A method for producing a culture medium or a culture auxiliary additive that modifies characteristics of hematopoietic stem cells, the method comprising:
preparing a cell population containing hematopoietic stem cells;
culturing the cell population containing hematopoietic stem cells in a medium having a configuration to be evaluated or a medium containing a culture auxiliary additive to be evaluated;
determining a configuration of the culture medium or the culture auxiliary additive using, as an index, an expression level of a marker containing one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in the cultured cells; and
producing a culture medium or a culture auxiliary additive that has the determined configuration.

15. The method according to any one of claims 5 to 14, wherein the cell population containing hematopoietic stem cells is cells derived from a living body or cells derived from cells having artificially acquired pluripotency.

16. Isolated or enriched hematopoietic stem cells or a cell population containing hematopoietic stem cells produced by the method according to any one of claims 5 to 7, wherein the cell population containing hematopoietic stem cells is cells derived from a living body or cells derived from cells having artificially acquired pluripotency.

17. A method for screening a compound inducing hematopoietic stem cells, the method comprising:
preparing a cell population;
treating the cell population with a test compound; and
selecting a candidate compound using, as an index, an expression level of a marker containing one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof, the marker being expressed in the cells treated with a candidate compound.

18. A composition or kit for identifying, detecting, isolating, or enriching hematopoietic stem cells, the composition or kit comprising a reagent for quantifying or detecting an expression level of one or more selected from CD10, CD111, CD112, CD131, CD143, CD244, CD275, CD328, CD62L, GPR56, CD133, CD36, CD370, CD84, CD326, CD114, CD116, CD135, CD172a/b, CD200, CD226, CD245, CD31, CD317, CD40, CD41, CD61, CD93, Notch1, CD352, Siglec-10, CD45RO, CD11c, CD52, CD318, CD49f, c-Kit, and CD48, or one or more selected from a combination of CD48 and CD34, a combination of CD48 and c-Kit, and a combination of CD48, CD34, and c-Kit, or a combination thereof.

19. A method for identifying a combination of physical, chemical, and biological characteristics obtained by analyzing hematopoietic stem cells identified, isolated, or enriched by the marker according to claim 1 or 2, the isolated or enriched hematopoietic stem cells or the cell population containing hematopoietic stem cells according to any one of claims 3, 4, and 7, or isolated or enriched hematopoietic stem cells or a cell population containing hematopoietic stem cells produced by the method according to claim 5 or 6.

20. A method for producing a classifier for identifying hematopoietic stem cells using, as an index, a combination of physical, chemical, and biological characteristics obtained by analyzing hematopoietic stem cells identified, isolated, or enriched by the marker according to claim 1 or 2, the isolated or enriched hematopoietic stem cells or the cell population containing hematopoietic stem cells according to any one of claims 3, 4, and 7, or isolated or enriched hematopoietic stem cells or a cell population containing hematopoietic stem cells produced by the method according to claim 5 or 6.

21. A drug for preventing or treating a disease or disorder ameliorated or prevented by administering hematopoietic stem cells, the drug comprising hematopoietic stem cells identified, isolated, or enriched by the marker according to claim 1 or 2, the isolated or enriched hematopoietic stem cells or the cell population containing hematopoietic stem cells according to any one of claims 3, 4, and 7, or isolated or enriched hematopoietic stem cells or a cell population containing hematopoietic stem cells produced by the method according to claim 5 or 6.

22. The drug according to claim 21, wherein the disease or disorder ameliorated or prevented by administering the hematopoietic stem cells is metachromatic leukodystrophy (MLD), Gaucher's disease, Fabry disease, mucopolysaccharidosis, HIV, ADA-SCID, X-SCID, or thalassemia.

23. The drug according to claim 21, wherein the disease or disorder ameliorated or prevented by administering the hematopoietic stem cells is a chronic granulomatous disease (CGD) or adrenoleukodystrophy (ALD).
